# EUROPEAN PATENT SPECIFICATION

(11) **EP 0 670 848 B1**
(45) Date of publication and mention of the grant of the patent: **04.02.2004**
(21) Application number: 92924278.2
(22) Date of filing: 04.11.1992
(51) Int. Cl.: C12N 1/21, C12N 1/00, C12N 15/12, C12P 21/00, C07K 14/78

(54) **HIGH MOLECULAR WEIGHT COLLAGEN-LIKE PROTEIN POLYMERS**
KOLLAGENARTIGE PROTEINPOLYMERE MIT HOHEN MOLEKULARMASSEN
POLYMERES PROTEIQUES ANALOGUES AU COLLAGENE, ET D'UNE MASSE MOLECULAIRE ELEVEE

(30) Priority: 12.11.1991 US 791960
(43) Date of publication of application: 13.09.1995
(73) Proprietor: PROTEIN POLYMER TECHNOLOGIES, INC., San Diego, CA 92121 (US)
(72) Inventor: CAPPELLO, Joseph, San Diego, CA 92122 (US); FERRARI, Franco, A., La Jolla California 92037 (US)
(74) Representative: Brewster, Andrea Ruth
(86) International application number: PCT/US1992/009485
(87) International publication number: WO 1993/010154

(56) References cited:
- WO-A-88/03533
- WO-A-88/05082
- WO-A-90/05177
- WO-A-93/10231
- Ann. N. Y. Acad. Sci., Volume 460, issued 30 December 1985, E.J. MILLER, "The Structure of Fibril-Forming Collagens", pages 1-13, especially page 4.
- Gene, Volume 80, issued 1989, I. GOLDBERG et al., "Cloning and Expression of a Collagen-Analog-Encoding Synthetic Gene in Escherichia coli", pages 305-314, entire document.

## Description

### INTRODUCTION

### Technical Field

The field of this invention is the production of collagen-like polymers using recombinant techniques.

### Background

Collagen, a naturally occurring protein, finds wide application in industry. Chemically hydrolyzed natural collagen can be denatured and renatured by heating and cooling to produce gelatin, which is used in photographic and medical applications, among other applications. The chain property of collagen responsible for this phenomenon is its ability to spontaneously form interchain aggregates having a conformation designated as a triple helix. The helices are stabilized by weak interactions between chains, arising from the close proximity of the peptide backbone at locations every third residue occupied by glycine and kinks provided by proline and hydroxyproline at the two positions between glycine. The geometry of the three kinked chains allows for hydrogen bonding within the triple helix. The structure is loose and is readily accessible to interaction with water, small organic and inorganic molecules, other proteins, and cells. Although collagen consists of many different amino acid sequences, one of the more structurally stable segments exists at the amino and carboxyl terminal ends of the processed collagen Type I chains. These ends consist to a large degree of the repeating tripeptide sequence GPP (the second P is often hydroxylated).

By contrast with natural forms of collagen, recombinantly-produced collagen-like polymers may consist exclusively of a single repeating tripeptide sequence selected from a wide variety of GXY sequences, where X and Y can be any amino acid, whether derived from known natural sequences or not. Collagen-like polymers can also consist of different tripeptide sequences, which are repeated as blocks in the final polymer. Dissimilar blocks can also be used in a repeating fashion to create block copolymers in order to provide additional chemical or biological functionality.

With the advent of recombinant technology, the opportunity arose to produce collagen-like polymers, where the advantageous properties of collagen could be selectively retained, while new capabilities and characteristics could be introduced. The uniqueness of collagen, the repetitive tripeptide, is a challenge for recombinant technology in light of the high repetitiveness of the sequence and the frequent utility of the amino acids glycine and proline in the composition. Genes encoding proteins with high levels of glycine and proline are by necessity composed of high levels of the nucleotides guanidine and cytidine in self complementary sequences. Thus, as one synthesizes the gene, there is substantial opportunity for strands to loop out, single-stranded DNA to be excised, recombination events to occur which can result in loss of segments of the gene, and inefficient transcription and/or translation. Thus, there is substantial interest in developing techniques and compositions which provide the advantageous properties of collagen, while at the same time allowing for stable expression of high molecular weight collagen-like proteins.

### Relevant Literature

WO 88/05082 and Goldberg, et al. *Gene* (Amst.) 80:305-314 (1989) describe the preparation of relatively low molecular weight collagen analog proteins. See also PCT/US87/03360 and PCT/US 89/05016 which describe structural protein polymers.

### SUMMARY OF THE INVENTION

High molecular weight collagen-like polymers, as defined in the claims, are provided by synthesizing genes, having glycine at every third position, and not more than about 60% of the amino acids present between the glycines are proline. The genes are prepared by synthesizing sequences having a plurality of tripeptide units, ligating the sequences together to provide multimers, cloning the multimers, and finally joining the multimers to provide for genes expressing high molecular weight collagen-like proteins, for the most part, in unicellular organisms.

### DESCRIPTION OF THE SPECIFIC EMBODIMENTS

Methods and compositions as defined in the claims are provided for producing high molecular weight collagen-like polymers, where glycine is retained as the first amino acid of a triad repeating unit and the other two amino acids are varied, so as to reduce the proline content of the polymer between the glycines to less than 60%, usually to less than about 40%, more usually less than 30%, based on amino acids in the collagen-like protein. The polymers may be varied in a variety of ways, having a single repeating unit, having alternating repeating units, or having blocks of different repeating units. Usually the polymers will have at their 5' and 3' termini sequences which will contribute fewer than about 20%, more usually fewer then about 10%, of the amino acids of the polymers. Genes are synthesized for encoding the collagen-like polymers, inserted into appropriate expression vectors, which are then introduced into expression hosts. The resulting collagen-like polymers may then be isolated, purified, and used in a variety of ways.

The polymers of this invention will be characterized by having a molecular weight of at least about 30 kD (kiloDaltons), more usually at least about 40 kD and preferably at least about 50 kD, usually not exceeding about 150 kD, more usually not exceeding about 125 kD and frequently not exceeding about 100 kD. The polymers will be further characterized in, being like collagen, providing helices, capable of denaturation and renaturation, forming gels, etc. The polymers will preferably have a major proportion of tripeptide triad sequences found in natural collagens, particularly mammalian collagen. The subject polymers may be modified in a variety of ways, by introducing various functional units, where depending upon the functional units, they may be a part of the design, providing for the uniform alternating glycine at the third position or interrupting the uniformity, or may be present at the end of a block of repetitive units. Any of the 20 amino acids may be present, although charged amino acids would usually be present in fewer than 30%, frequently in fewer than 15%, by number in a repetitive sequence.

The subject collagen-like polymers can be widely varied as to their repeating units, the pattern of repeating units, the insertion of interrupting sequences, and the like. At least 75 weight percent, usually at least 80 weight percent, more usually at least 85 weight percent, will be composed of triads (tripeptides with glycine as the first amino acid). Usually at least 50%, more usually at least 80% of the triads will be found in natural collagen, particularly mammalian collagen. The number of triads will usually be at least about 100, more usually at least about 150, and not more than about 500, usually not more than about 400.

The compositions of this invention will be described by formuli, where each capital letter will indicate an amino acid, where the one letter description for an amino acid will indicate that particular amino acid and where a letter which is not within the one letter description will be used to symbolize any amino acid or a designated group of amino acids. The superscripts will indicate that where there are a plurality of triads, the letter symbolic of amino acids may symbolize the same or different amino acid in each of the triads. Subscripts will always intend the number of repeats of the symbol or formula to which the subscript relates.

For the most part and subject to the requirements of the claims, the polymers will be characterized by having as a motif present at least twice in the collagen-like polymer, either contiguous or non-contiguous, a sequence having the following formula:

((GXO)ₙZ)ₘ

wherein G is glycine; X and O are any amino acids except that X and O are selected to have a proline content of the repetitive sequences of the polymer, usually the entire polymer, of less than about 45% by number, usually of less than about 40%, and may be less than 30%, and greater than about 10%, usually greater than about 15%;
Z has from 0 to 50 amino acids, more usually from 0 to 30 amino acids, frequently from 0 to 15, and will usually be a sequence having functional capability and will be other than repetitive GXO;
n will be at least 4 and not more than about 100, more usually not more than about 75, frequently not more than about 50; and
m will be at least 1, may be 2, usually at least about 3 and not more than about 20, usually not more than about 12, particularly m is smaller as n becomes larger.

The repetitive sequence of triads will be at least 60%, usually at least 80% by weight of the polymer.

The total number of different triads in the motif will be at least 3, and usually not more than about 24, more usually not more than about 16 and frequently not more than about 12. The total number of different amino acids in the motif will usually be not more than about 15, more usually not more than about 10. The number of triads including proline will be at least 40 number percent, more usually at least 60 number percent and not more than about 98 number percent. Although all of the triads may include one proline, preferably fewer than 85 number percent, usually fewer than 75 number percent, will include proline.

One type of collagen-like polymer will for the most part have the following motif, as a repetitive motif.

((GX^{x}O^{o})ₙ¹Z^{z})ₘ¹

wherein GXO are as defined previously; x and o are the same number and will be in the range of 2 to 10, usually 3 to 8 and often 4 to 6, particularly 2, 3, 5 or 10, which intends that there will be up to and including that many different triads present in the sequence; Z has been defined previously; z is 1 to m¹ usually not greater than 3, frequently 1 to 2, preferably 1 where z intends different Z groups, similar to the definition of x, and m¹ is at least 1, may be 2, usually at least about 3, and not more than about 20, usually not more than about 12, particularly as n¹ becomes larger; and n¹ is at least about 2 and not more than about 100, usually not more than about 75, more usually not more than about 50, generally not more than about 30.

For example, where x is 3, Z is O amino acids, and m' is 1, one would have the formula:

((GX¹O¹)(GX²O²)(GX³O³))ₙ¹

where X¹ is a particular amino acid, X² could be the same or different amino acid, etc., and similarly for O; and
n¹ will generally be at least about 2, more usually about 4 , and not greater than about 33, usually not greater than about 25.

One technique for synthesis of the subject compounds lends itself to a particular motif of the formula:

{(α)ₐ(β)_{c}(α)ₐ¹}_{g}

wherein:
α has from 1 to 9 triads, usually 2 to 6 triads, preferably 2 to 4 triads, usually at least 1 triad having a proline, more usually at least 2 triads having a proline;
β has from 1 to 24 triads, usually 2 to 18 triads, more usually 3 to 9 triads;
e will be at least 3, usually at least 6, and not more than about 50, usually not more than about 36;
g is at least 1 and not more than about 20, usually not more than about 10, more usually not more than about 8;
a and a¹ may be the same or different; a will be at least 1 and not more than 6, usually not more than about 3, except that a or a¹ may be 0.

It will be understood that the greater the number of triads present, the lower the number of repeats required to provide the desired molecular weight.

In a preferred embodiment, these extended motifs will have, for the most part, the following formula:

(GU^{u}B^{b})ₚ((GX^{x}O^{o})ₙ¹(GU^{u}B^{b})ₚ¹Z^{z})ₘ¹

G X^{x}, O^{o}, Z^{z}, m¹ and n¹ have all been defined previously:
U and B are any amino acids, frequently there being a proline present in at least one of the triads (see also the definitions for X and O, which are included herein);
u and b have analogous meaning to x and o, indicating the same or different amino acids in the different triad (tripeptide) units; and
p and p¹ will be 1 to 6, more usually 2 to 4, and the two p's may be the same or different, usually the same except that p may also be 0;

The amino acids of particular interest other than glycine will include alanine (A), isoleucine (I), leucine (L), valine (V), serine (S), threonine (T), asparagine (N), glutamine (Q) , lysine (K), arginine (R), aspartic acid (D), glutamic acid (E), histidine (H), and proline (P), preferably there being at least one of a neutral polar amino acid, e.g., S or T, or a neutral aliphatic amino acid, e.g., A, I, L, or V. Usually, the aromatic hydrophobic amino acids will be avoided, e.g. , F, Y and W, and the aliphatic hydrophobic amino acids (L, V, I), will usually be used, if at all, when a proline is present. For structural considerations, triad sequences containing proline, alanine, valine, serine, threonine, glutamine, or asparagine in the X or O positions form more stable triple helices. Increased stability is also conferred by triads containing hydroxyproline in the O position. Triads containing tryptophan and tyrosine are usually not utilized. For higher order assembly of triple helices, triads containing glutamic acid or aspartic acid at either the X or O positions, but especially at the X position, and triads containing lysine or arginine, at either the X or O position, but especially at the O position are preferred.

Of particular interest is where at least one triad has proline at a second position, more preferably that two triads have proline at second positions or at least one triad has proline at a third position, sometimes having two triads having proline at a third position, there frequently being at least two triads having a proline at either the first and second position. For the most part, at least 30%, more usually at least 50% of the triads in the motifs will have proline at a second or third position and there may be some triads with proline at both positions, usually not more than 35 number percent.

The choice of triads utilized in a recombinant collagen like polymer are chosen in order to affect properties such as helix stability, hydration, solubility, gel point, biodegradation, and immunogenicity. For example, in order to minimize immunogenicity, triads are utilized consisting of amino acids with simple side chains such as glycine, alanine, serine, valine, and proline. When more complex amino acids are required, they can be incorporated by utilizing hexapeptide, nonapeptide, or longer triad sequences from natural collagen chains that contain these amino acids.

To a great extent, many of the polymers of the subject invention may be depicted by the following formula:

J¹ᵣ((GU^{u}B^{b})ₚ²(GX^{x}O^{o})ₙ¹(GU^{u}B^{b})ₚ²)Z₁)ₘ¹)J²ᵣ

wherein all of the symbols have been described previously, except for:
J¹ and J² which are the same or different and are amino acid sequences of from about 1 to 40 amino acids, more usually from about 1 to 30 amino acids, and may be any sequence of convenience;
the two r's are the same or different and are 0 or 1;
the two p²'s are the same or different, and are 0 or p¹.

A variety of repetitive sequences defining the motifs may be illustrated by the following sequences:

GAPGPAGPK; GAPGPAGPPGAH; GSPGAPGPA; GAPGPAGSRGDPGPA;
GANGAPGPAGPAGAPGPP; GAQGPAGPGGAPGPAGPG; GAPGPAGAS;
GPAGAPGSRGDPGAPGPP; GVSGPRGPAGAPGPP; and GAQGPAGPG

Triads of particular interest include GAP, GPA, GPP, GAS, GPG, GPS, GAQ, GSP, GSQ, GLQ, GPR, GPK, GAK, GAR, GER, GDR, GEP, GDA, GAH and GEA, where combinations of these triads with other triads are of particular interest, there being at least about 30 number percent, usually at least about 50 number percent of the triads, more usually at least about 60 number percent, frequently at least 80 number percent, coming within the indicated triads. In addition, while the number of triads in any one unit may vary widely, desirably the number will be 3, 5 or a multiple thereof.

Sequences defining the GUB sequences include:

GAHGPAGPK; GAHGPAGPR; GAVGAPGPK; GPAGAPGEP; and
GVSGPRGAP

The genes may be synthesized with the method described in PCT/US87/02822. Particularly, the genes are synthesized in accordance with the following protocol. Relatively short oligonucleotide strands are synthesized generally coding for at least 12 amino acids, and usually not more than about 60 amino acids. The complementary strands are annealed, cloned, and sequenced to establish that the correct sequences have been obtained. The ends may be blunt or staggered, usually staggered. The sequences are then multimerized and ligated and inserted into a cloning vector. After transforming into an appropriate host, the clones are analyzed for the oligomerization of the units and appropriate sized units are selected and used for expression. When flanking repetitive units of different sequences are desired, the sequence encoding the motif may be inserted at an appropriate restriction site between the flanking repetitive units, so that subsequently the gene may be excised with the appropriate flanking units. The resulting sequence encoding the motif with the flanking units may then be excised, oligomerized, and, as before, cloned and identified as to having the desired sequence and size.

The subject proteins are produced by transforming an appropriate unicellular host, such as E. coli, B. subtilis, yeast, S. pombe, S. cerevisiae, etc., fungi, Neurospora, Aspergillus, etc. A wide variety of expression vectors have been described in the literature and need not be exemplified here. The expression vector may provide for an inducible or constitutive promoter, whereby the subject proteins may be continuously expressed or expressed only after induction. Numerous expression vectors have appropriate promoters, termination sequences, and polylinkers between the initiation and termination sequences, so that one may conveniently insert the desired gene into the polylinker to be under the transcriptional control of the promoter. In addition, the vectors may have one or more markers, which allow for selection in the transformed host. The vector may also allow for extrachromosomal maintenance or integration into the genome. The markers may provide for resistance to a toxin, e.g. an antibiotic, or complementation of an auxotrophic host to prototrophy. The choice of expression vector will depend upon on a number of factors, such as the host, economy, ease of manipulation, and the like.

Once the expression vector has been cloned, it will normally be analyzed to ensure that the gene is present and has not undergone any modification. The expression host can then be grown up in an expression media and after sufficient time, the cells lysed and the protein isolated. In some cases, the protein will be relatively insoluble and, therefore, may be separated from cellular debris by various convenient means, e.g. centrifugation. Depending on the degree of purity, the protein may be used as is, or may be further purified by extraction using a variety of solvents to extract out the impurities, while leaving the desired product in insoluble form. If desired, dilute acid solution may be used for solubilizing the protein and then precipitating the protein by dialysis. The conditions employed will vary with the particular protein produced, its solubility, the nature of the host, the nature of the contaminants, the ultimate use, and the like.

Small peptides may be prepared which include small segments of the collagen-like polymers, e.g., 15 to 36 amino acids. These segments may be used as haptens, by conjugating the segment to an appropriate immunogen, and the resulting immunogen used for production of antisera or monoclonal antibodies specific for the sequence. Thus, antibodies may be produced which specifically bind to the collagen-like polymers of the subject invention. Depending on the solubility of the subject polymers, the antibodies may be used for affinity purification, identification of the polymers on Western blots, or non-covalently crosslinking of the polymers to produce novel structures and the like.

The subject compositions may find use for a variety of applications, for structural units, in medicine, and the like. The subject collagen-like polymers are useful in the formulation of materials, such as fibers, membranes, films, coatings, hydrogels, colloid suspensions and molded articles, imparting unique characteristics to these materials and to a variety of surfaces from either naturally occurring or synthetic sources, the characteristics usually being associated with water absorption, biocompatibility, or interaction with non-protein compounds of either organic or inorganic nature, *e*.*g*. silver halide, dyes, *etc.* Coatings of 1 to 100 mil may find use, with adherency to a variety of surfaces from naturally occurring and synthetic sources. Distinctive properties of these polymers are their thermal reversible gelation at specific temperatures, their inherent biocompatibility and their bioresorption. Therefore, the subject polymers may find application in photographic films, as wound dressings, allowing for neovascularization, eye applications, bone cements, matrices for artificial organs, membranes, coatings for growing cultures, implantable or injectable drug delivery systems, and the like.

Furthermore, since natural collagen is cross-linked it cannot be redissolved for spinning, shaping, etc. without digestion and molecular weight reduction. Thus, the collagen-like polymers retain the desired collagen characteristics and are capable of being processed.

The following examples are offered by way of illustration and not by way of limitation.

### EXPERIMENTAL

### Methods for the Production of High Molecular Weight Collagen-Like Protein Polymers

### Example 1

### DNA Preparation Methods

### 1. Preparation of plasmid DNA from E. coli.

A. Small scale. Plasmid DNA was prepared from 1.5 ml cultures by either the boiling procedure or the alkaline lysis method (Maniatis et al., Molecular Cloning: A Laboratory Manual, Cold Spring Harbor Laboratory, Cold Spring Harbor, (1982).
B. Large scale. A plasmid-carrying strain was grown overnight in 1 liter of Luria broth with the appropriate antibiotic. The cells were collected by centrifugation at 10,000xg for 5 minutes and resuspended in 10 ml of ice cold TE (10 mM Tris-HCl pH 8, 1 mM EDTA). The cells were centrifuged again, resuspended in 4 ml of TES (TE and 25% w/v sucrose) and homogenized by vortexing. The samples were kept on ice for the following steps. Lysozyme (1 ml of 10 mg/ml) was added to the cell suspension and incubated for 5 minutes before the addition of 2 ml of 0.5 M EDTA pH 8. After 10 minutes incubation, 50 ml of proteinase K (40 mg/ml) were added followed 10 minutes later with 15 ml of lysing buffer (0.1% Triton X-100, 1 mM EDTA, 50 mM Tris-HCl pH 8). After 15-20 minutes, the cell lysate was centrifuged at 35,000xg for 90-120 minutes. The supernatant (19.8 ml) was transferred to a plastic tube with 20 mg of CsCl and 400 µl of ethidium bromide (10 mg/ml). After dissolution, the mixture was divided into two polyallomer ultracentrifuge tubes, sealed with heat and centrifuged in a Beckman Ti 65 motor at 60,000 rpm for 24 hours. The lower plasmid DNA band was removed from the tube with a hypodermic needle. The ethidium bromide was extracted three times with an equal volume of NaCl-saturated isopropanol. Two volumes of H₂O were added to the DNA solution, and then the DNA was precipitated with ethanol.

### 2. Deproteinization.

Phenol extraction was performed on a convenient volume of DNA sample, typically between 100 µl to 10 ml. The DNA sample was diluted in 0.01 M Tris-HCl pH 7.5, 1 mM EDTA and an equal volume of water-saturated phenol was added. The sample was vortexed briefly and placed on ice for 3 minutes. After centrifugation for 3 minutes in a microfuge, the aqueous layer was removed to a new tube and extracted once with an equal volume of chloroform:isoamylalcohol (24:1).

### 3. Ethanol precipitation.

DNA in an aqueous buffer was concentrated by ethanol precipitation. To the DNA sample was added 1/10 volume of 3 M sodium acetate pH 7.5 and 2-3 volumes of cold ethanol. The DNA was precipitated for 30 minutes at -70°C or overnight at -20°C and then pelleted by centrifugation in the microfuge for 15 minutes at 4°C. The pellet was washed once with 200 µl of cold 80% ethanol and precipitated again for 10 minutes at 4°C. After air drying or lyophilization, the pellets were resuspended in the appropriate buffer.

### 4. Phosphatase treatment of DNA.

A. Phosphatase treatment of DNA was performed by adding 1 µl (25 units) of calf intestinal phosphatase (Boehringer Mannheim) directly to the restriction enzyme digestion reaction and continuing the incubation for 30 minutes at 37°C. The phosphatase was inactivated for 60 minutes at 65°C prior to deproteinization by phenol extraction.
B. Phosphatase treatment of DNA was also performed by resuspending ethanol precipitated DNA from the restriction enzyme digest in 20 mM Tris-HCl pH 8.0, 10 mM MgCl₂ to a final DNA concentration of 20 µg/ml. Shrimp Alkaline Phosphatase was added at 2 U/µg of DNA and the mixture was incubated at 37°C for one hour, heat inactivated for 20 minutes at 65°C and then passed through a Probind filter (Millipore) by microfuging for 30 minutes at 12,000 RPM. The DNA was then ethanol precipitated and resuspended in suitable buffer.

### 5. Phosphorylation of DNA.

Phosphorylation before annealing was performed by using Polynucleotide Kinase 3'-phosphatase-free (Boehringer Mannhaim) . The reaction was carried out at 37°C for 30 minutes in a 50 µl reaction volume containing: 12.5 µg DNA, 5 µl 10x kinase buffer (0.5 M Tris pH 7.5, 10 mM Spermidine, 0.1 M MgCl₂, 150 mM DTT, 1 mM EDTA), and 2 µl Polynucleotide Kinase (10 u/µl). After phosphorylation, salts and glycerol were removed from the DNA strands using a Bio-Spin 6 column (BioRad) equilibrated in TEAB.

### 6. Fill-in reaction with DNA polymerase 1.

DNA was resuspended in buffer containing 50 mM Tris-HCl pH 7.4, 50 mM KCl, 5 mM MgCl₂, and 400 mM each of the four deoxynucleotide triphosphates. Ten units of Klenow DNA polymerase (BRL) were added, and the reaction was allowed to proceed for 15 minutes at room temperature. The DNA was then phenol extracted and ethanol precipitated.

### 7. Digestion with restriction endonucleases.

DNA was digested with restriction endonucleases (REN) in 1 x "AA" buffer [10 x AA buffer is 330 mM Tris-acetate, pH 7.9, 660 mM potassium acetate, 100 mM magnesium acetate, 50 mM dithiothreitol (DTT) and 1 mg/ml bovine serum albumin (nuclease free)]. Whenever possible, the concentration of DNA was kept below 1 µg/25 µl. Incubation was at 37°C for 1-4 hours for most restriction endonucleases except for Ball, Banl and Nael digestions which were incubated overnight.

### 8. Analytical agarose gel electrophoresis of DNA.

To DNA samples for gel analysis was added 0.2 volumes of loading buffer (5 x electrophoresis buffer, 0.01% bromphenol blue dye, 50 mM EDTA, and 50% glycerol). Then the samples were loaded into lanes of a horizontal submerged electrophoresis unit containing a 1.0% (w/v) agarose gel. The electrophoresis buffer was either 1 x TAC or 1/2 x TBE. The 1 x TAC is 40 mM Tris-base, 10 mM EDTA, adjusted to pH 7.8 with acetic acid. The 1/2 x TBE is 0.045 M Tris-base, 0.045 M boric acid, 1 mM EDTA, pH 8. The gel was run at 40-50 V for 18 hours, then removed and stained with 0.5 g/ml ethidium bromide for 30 minutes. The DNA bands were visualized on a long wavelength UV transilluminator.

### 9. Preparative agarose gel electrophoresis.

The procedures and materials are the same as for the analytical agarose gel electrophoresis. The only difference is the use of low melting point (LMP) agarose, ranging in concentration from 0.5 to 2.5% (w/v) depending on the size of the DNA fragment to be purified. DNA restriction fragments were excised from the LMP agarose gels after visualization with ethidium bromide. For agarose ligation the buffer used was 1 x TAE (50 mM Tris Acetate).

### 10. NACS purification.

Gel fragments containing DNA were melted at 70°C for 5 minutes and diluted approximately 5 fold with TEI (10 mM Tris-HCl pH 7.5, 0.2 M NaCl). The gel solution was applied to a NACS column (BRL). The column was washed with 5 ml of the same buffer. The bound DNA was eluted with 300 µl of either TE2 (10 mM Tris-HCl pH 7.5, 1.0 M NaCl) for DNA fragments smaller than 1000 bp or TE3 (10 mM Tris-HCl pH 7.5, 2 M NaCl) for larger fragments. The eluted DNA was concentrated by ethanol precipitation.

### 11. DNA ligation.

Reactions for ligating cohesive ends contained: 1 µg DNA, 1 x AA buffer (see step 6, above) 1 mM ATP and 20 units of T4 DNA ligase (BRL) in a 20 µl final reaction volume. The ligation was allowed to proceed for 16-18 hours at 15°C or 1-2 hours at room temperature. For blunt-ended ligations the reactions contained 1 µg DNA, 25 mM Tris-HCl pH 7.5, 5 mM MgCl₂, 5 mM DTT, 0.25 mM spermidine, 200 ng BSA, 1 mM hexamine cobalt chloride (HCC), 0.5 M ATP and 400 units T4 DNA ligase (NEB) in a 20 µl reaction volume. The ligation was allowed to proceed for 30 minutes to 1 hour at room temperature.

### 12. Agarose DNA ligation.

The agarose was melted at 65°C, the temperature was then lowered to 37°C and ligation buffer (5X= 100 mM Tris-HCl, pH 7.5, 50 mM MgCl₂, 50 mM DTT, 1 mM ATP) was added; the tube was then placed at room temperature and ligase was added (1000 units T4 DNA ligase (NEB), the reaction volume was usually 50 µl. The reaction was incubated at 15°C for 16-18 hours.

### mRNA Methods

### 1. Preparation of mRNA.

mRNA was prepared using a modified procedure described by Summers, W.C. (Anal. Biochem. 33:459-463 (1970). Cells were grown at 30°C or 42°C in LB medium supplemented with kanamycin (50 µg/ml). 10 ml of cells at OD₆₀₀ 1.0 were spun and the cell pellet was resuspended in 10 ml of protoplasting buffer (15 mM Tris-HCl pH 8.0, 0.45 M sucrose, 8 mM EDTA); 80 µl of lysozyme at 50 mg/ml were added and the mixture was then incubated on ice for 15 minutes. The cell suspension was spun for 5 minutes at 7,000 RPM in an SS-34 rotor using an RC-5B centrifuge. The pellet was resuspended in 0.5 ml of lysing buffer (10 mM Tris-HCl pH 8.0, 10 mM NaCl, 1 mM Na-Citrate, 1.5% w/v SDS) and 15 µl of DEPC. The suspension was mixed gently and then transferred to a 1.5 ml Eppendorf tube, incubated at 37°C for 5 minutes and then chilled on ice. 250 µl of saturated NaCl (40% w/v) were added, mixed gently, and the incubation, on ice, was prolonged for an additional 10 minutes. The slurry was spun at 4°C for 15 minutes. The supernatant was placed in two 1.5 ml tubes and 1 ml of 100% ethanol was added to each. The RNA was precipitated in the cold and then spun at 4°C for 20 minutes. The pellets were rinsed in 70% ethanol and dried. The RNA was then resuspended in 0.1 ml H₂O and OD₂₆₀ and OD₂₈₀ were taken to measure the recovery and purity of the RNA. The average recovery of total RNA (5% mRNA, 95% tRNA + rRNA), from 10 ml of growing cells, was between 0.5 and 1.0 mg.

### 2. Northern blot analysis.

The RNA, purified as described above, was run on agarose gel and transferred to nitrocellulose following the procedure described in Maniatis et al., Molecular Cloning: A Laboratory Manual, Cold Spring Harbor Laboratory, Cold Spring Harbor (1982), pp 202-203, using 10X SSC as transfer buffer. The ECL gene detection system kit (Amersham) was used for labelling of the probe, hybridization conditions and detection. The procedures were executed as described in: ECL gene detection system RPN2101, version 2, Amersham International plc.

### Bacterial Transformation Methods

### 1. Preparation of transformation-competent E. coli cells.

A culture of 200 ml of sterile L broth was inoculated with a small loopful of E. coli cells. This was incubated with shaking at 37°C until the OD₆₀₀ was approximately 0.5. The culture was placed on ice for 10 minutes and centrifuged at 6,000xg for 10 minutes. The cell pellet was resuspended in 100 ml of ice-cold 0.1 M MgCl₂, kept on ice for 30-40 minutes and centrifuged again. The pellet was resuspended in 2 ml of ice-cold 100 mM CaCl₂, transferred to a sterile test tube and incubated on ice for 24 hours. The competent cells were then aliquoted and stored at -70°C.

### 2. Transformation of E. coli.

An aliquot of frozen competent cells was thawed on ice. To 50 µl of cells, 0.1 to 1 µg of DNA was added and the mixture was incubated on ice for 30 minutes. The tube was removed from ice and placed in a 42°C bath for 2 minutes. L broth (1 ml) was added and the transformation mix incubated with shaking at the desired temperature (usually 30°C or 37°C) for 2 hours. Then one-tenth of the transformation was plated on L broth plates containing the appropriate antibiotic and, when necessary, XGAL and IPTG were added.

### Antibody Production, Protein Chemistry and Electrophoresis of Proteins

### 1. Preparation of antibody to artificially synthesized Peptides.

A synthetic peptide of sequence GAHGPAGPKGAHGPAGPKGAPGPAGPP-GAPGPAGPP (DCP-C₂A₂) was coupled to keyhole limpet hemocyanin for use as an immunogen. The material was sent to Antibodies, Inc. for preparation of antibodies in rabbits. Peptide conjugates at a concentration of 1 mg/ml in complete Freund's adjuvant were used to immunize rabbits at day 0. Animals were re-injected with antigen in Freund's incomplete adjuvant at day 30 and titered at day 60. Positive sera was detected using a microtiter RIA using the synthetic peptide as antigen. Kagen and Glick (1979), in Methods of Radioimmunoassay, Jaffe and Berman (eds.), Academic Press, p 328. Antisera was obtained that reacted with synthetic peptides of the DCP 1 and DCP 2 sequences.

Following the procedure described above an additional peptide was synthesized having the sequence (GAPGPAGPPGSRGDPGPP)₂(DCP-(AB)₂), which was also coupled to keyhole limpet hemocyanin for use as an immunogen. Polyclonal antisera were then prepared as described above, which bound to the synthetic peptide of the DCP3 sequence.

Following the procedure described above an additional peptide was synthesized having the sequence (GAPGAPGSQGAP-GLQ)₂ YMK which was also coupled to keyhole limpet hemocyanin for use as an immunogen. Polyclonal antisera were then prepared as described above, which bound to the synthetic peptide of the CLP 3.1 sequence.

### 2. Polyacrylamide gel electrophoresis of proteins.

Approximately 10⁹ E. coli cells from growing cultures were pelleted by centrifugation at 10,000xg for 5 minutes. The cell pellets were resuspended in 100 to 500 µl of 2X sample buffer (100 mM Tris-HCl pH 6.8, 4% SDS, 10% β-mercaptoethanol, 60% glycerol or sucrose) and sonicated for 30 seconds using a Tekmar sonic disrupter. Samples were boiled for approximately 5 minutes and 20 to 100 µl of the cell lysates were loaded on an SDS-polyacrylamide gel (7.5 to 16% w/v) . The gels were prepared following the procedure of Laemmli (Nature, 27:80-685 (1970)). The proteins in the gels were stained with 2% Coomassie brilliant blue in 10% methanol, 7.5% acetic acid for 1 hour and destained in 10% methanol, 7.5% acetic acid overnight.

### 3. Protein expression analysis.

An overnight culture which had been grown at 30°C was used to inoculate 50 ml of LB media contained in a 250 ml flask. Kanamycin was added at a final concentration of 50 µg per ml and the culture was incubated with agitation ( 200 rpm) at 30°C. When the culture reached an OD₆₀₀ of 0.8, 40 ml were transferred to a new flask prewarmed at 42°C and incubated at the same temperature for approximately 2 hours. The cultures (30°C and 42°C) were chilled on ice and OD₆₀₀ was taken. Cells were collected by centrifugation and then divided in 1.0 OD₆₀₀ aliquots and used to perform western analysis using the appropriate antibodies.

### 4. Immunoblotting of proteins in gels.

After protein electrophoresis, one of the flanking glass plates was removed from the polyacrylamide gel. The gel surface was wetted with transfer buffer (25 mM Tris-HCl, 192 mM glycine, 20% methanol). A piece of nitrocellulose paper (Sartorius, SM11307) was saturated with transfer buffer and laid on the gel. Air bubbles between the filter and the gel were removed. The gel and nitrocellulose filter were placed in the transfer unit as specified by the manufacturer (Bio-Rad). Transfer was allowed to proceed at 200 mA for 3-4 hours. Then the nitrocellulose filter was removed and stained with Amido-Schwartz for 3 minutes (0.05% Amido black, 45% deionized H₂O, 45% methanol, 10% acetic acid) and destained in H₂O. The filter was incubated for at least 10 minutes at room temperature in "BLOTTO" (5% w/v nonfat dry milk, 50 mM Tris-HCl pH 7.4, 0.9% w/v NaCl, 0.2% w/v sodium azide). The filter was placed in serum appropriately diluted (1:50 to 1:500) in 0.5X Blotto (2.5% nonfat dry milk, 50 mM Tris-HCl pH 7.4, 0.9% NaCl, 0.2% sodium azide) and was gently agitated for approximately 16 hours at room temperature. The filter was washed for 1 hour with 5 changes of TSA (50 mM Tris-HCl pH 7.4, 0.9% NaCl, 0.2% sodium azide). The blot was placed in 15 ml of 0.5X BLOTTO solution containing 1 x 10⁷ cpm of ¹²⁵I-protein A and gently agitated for 2 hours at room temperature. The filter was washed for 2 hours with a minimum of 7 changes of TSA, rinsed once with deionized H₂O and air dried. The blot was covered with Saran wrap and autoradiographed.

### 5. Amino Acid Analysis.

Amino acid compositions were determined by the PTC derivatization procedure of Henrickson and Meredith (1984). Protein samples were hydrolysed with 5.7 N constant boiling HCl at 108°C for 24 hours in vacuo. After reaction with PITC, amino acid derivatives were detected at 254 nm by HPLC reverse phase chromatography using a Waters 100E system and a Supelco C18 column (4.6 mm x 25 cm) with a linear gradient of 0-50% acetonitrile in 0.1 M NH₄OAc pH 6.78 as a mobile base. Henrickson, R.L. and Meredith, S.C. (1984) Amino Analysis by Reverse Phase High Performance Liquid Chromatography., Anal. Biochem. 137:65-74.

### 6. Peptide Synthesis.

Synthetic peptides were prepared by solid phase synthesis on an Applied Biosystems Model 430A Peptide Synthesizer using the standard symmetric anhydride chemistry as provided by the manufacturer. The coupling yield at each step was determined by the quantitative ninhydrin procedure of Sarin et al., (1981). The synthetic peptide was cleaved from the solid support and amino acid blocking groups were removed using anhydrous HF (Stewart and Young, (1984)). Crude peptides were desalted by chromatography over Sephadex G-50. Sarin, V.K., Kent, S.B.H., Tam, J.P. and Merrifield, R.B. (1981) Anal. Biochem. 237:927-936. Stewart, J.M. and Young, J.D. (1984) Solid Phase Peptide synthesis, Pierce Chemical Company, Rockford, Il pp 85-89.

### Synthetic DNA Methods

### 1. In vitro DNA synthesis.

The N,N-diisopropyl phosphoramidites, controlled-pore glass columns and all synthesis reagents were obtained from Applied Biosystems, Foster City, California. Synthetic oligonucleotides were prepared by the phosphite triester method with an Applied Biosystems Model 381A DNA synthesizer using a 10-fold excess of protected phosphoramidites and 1 µmole of nucleotide bound to the synthesis support column. The chemistries used for synthesis are the standard protocols recommended for use with the synthesizer and have been described (Matteucci et al., J. Amer. Chem. Soc., 103:3185-3319 (1981)). Deprotection and cleavage of the oligomers from the solid support were performed according to standard procedures as described by McBride et al., Tetrahedron Letters, 24:245-248 (1983). The repetitive yield of the synthesis as measured by the optical density of the removed protecting group as recommended by Applied Biosystems (1984) was greater than 97.5%. The crude oligonucleotide mixture was purified by preparative gel electrophoresis as described by the Applied Biosystems protocols of November 9, 1984 (User Bulletin No. 13). The acrylamide gel concentration varied from 10 to 20% depending upon the length of the oligomer. The purified oligomer was identified by UV shadowing, excised from the gel and extracted by the crush and soak procedure (Smith, Methods in Enzymology 65:371-379 (1980)).

### 2. Sequencing of DNA.

DNA sequences were determined by the methods described in the following references: Maniatis *et al*., Molecular Cloning: A Laboratory Manual. Cold Spring Harbor Laboratory, Cold Spring Harbor, NY; Norrander *et al*., Gene (1983) 26:101-106; Sanger *et al*., Proc. natl. Acad. Sci. USA (1977) 74:5463-5467; Biggin *et al*., Proc. Natl. Acad. Sci. USA (1983) 80: 3963-3965; and Sanger *et al*., FEBS Letters (1978) 87:107-110.

Fragments containing the region of interest were cloned into the multiple cloning site of M13mp18 or M13mp19 (Maniatis et al., (1982), and Norrander et al., (1983)). Single-stranded DNA was prepared and sequenced by the primer extension method (Sanger et al., (1977) and Biggin et al., (1983)) using ³⁵S-deoxyadenosine 5'-(alpha-thio)- triphosphate (New England Nuclear) as label. In some cases, reverse transcriptase (Molecular Genetics) was used to extend the primer, using the dideoxy:deoxynucleoside triphosphate ratios utilized by Zagursky et al. (Gene Anal. Tech. (1985) 2:89-94). Deoxyadenosine triphosphate labeled with either ³²P or ³⁵S was used in these reactions. Compression artifacts which appeared in some G-C rich sequences were overcome by eliminating deoxyguanosine triphosphate from the G reaction, and using deoxyinosine triphosphate (P-L Biochemicals) at a final concentration of 37.5 µM instead. In the other mixes, the concentration of dideoxyGTP in the G reaction was 0.5 mM. All sequences were run on 6 or 8% polyacrylamide gels containing 8 M urea (Sanger et al., (1978)). Primers used for sequencing were purchased from P-L Biochemicals.

### 3. Dideoxy DNA sequencing of double stranded plasmid DNA.

Plasmid DNA was prepared as described previously (Preparation of plasmid DNA from E. coli, Small scale, Maniatis et al.). Primers were synthesized using a DNA synthesizer as described previously, and were annealed to the plasmid DNA following the procedure described above for M13 sequencing. The sequencing reactions were done using Sequenase (United States Biochemicals) and the conditions were as recommended by the supplier. All sequences were run on polyacrylamide gels as described above.

### Example 2

**TABLE 1**

| STRUCTURE OF DCP 1, 2 & 3 | |
|---|---|
| DCP1 | (C₂A₂₄C₂)₄ |
| DCP2 | (C₂A₁₂C₂)₈ |
| DCP3 | (C₂(AB)₁₂C₂)₄ |

where:
A = GAPGPAGPP
B = GSRGDPGPP
C = GAHGPAGPK

### DNA DESIGN

Due to the complexity of the DCP polymer structures the design of the gene monomers was as follows:
1. design and synthesis of C₂ units (5' and 3')
2. design and synthesis of A units
3. design and synthesis of AB units

### PLASMID pPT 0134 CONSTRUCTION

The acceptor vector PPT 0134 was designed and constructed specifically to accommodate the construction requirements of the DCP polymer genes. This vector contains two recognition sites for Fokl REN in its MCS (multiple cloning site).

Two oligonucleotide strands containing these sites were synthesized and purified as described in Example 1.

Fokl                                    Fokl   Scal

0.A) 5' - GTGCTGCGGATGCTCGAGATGGTGCATGCATGTACATCCGAGTACTTCGAT

O.B) 3' - ACGCCTACGAGCTCTACCACGTACGTACATGTAGGCTCATGAAGCTA

After annealing, the two oligonucleotide strands were ligated with pSY 937 (see patent application number PCT/US87/02822) which had been digested with Banl and EcoRV RENs. The product of the ligation mixture was transformed into E. coli and selected on bacterial plates containing the antibiotic chloramphenicol. Plasmid DNA from individual colonies was analyzed on agarose gel electrophoresis after digestion with Scal and Stul RENs. One plasmid pPT 0124 contained the expected DNA fragment.

The new MCS was then moved to plasmid pSY 1367. This plasmid is a derivative of pSY 1299 (see patent application number PCT/US87/02822). Plasmid pSY 1299 was digested with Ncil REN and the large DNA fragment was purified by agarose gel electrophoresis and NACS purification. The purified DNA fragment was treated with DNA Polymerase (see Example 1), ligated, then digested with Fokl prior to transformation in E. coli strain HB 101. Plasmid DNA from single colonies was purified and analyzed by restriction digests. One plasmid, pSY 1366, was found to be correct and lacking the only Fokl site present in pSY 1299.

Two oligonucleotide strands were synthesized and purified as described in Example 1:

(Banll)                    Fokl

1.A) 5' -     CTACATGTGTTACACATCCCGTGC

1.B) 3' - CCGAGATGTACACAATGTGTAGGGCACG

Oligonucleotide strands 1.A and 1.B were annealed and ligated with the DNA of plasmid pSY 1366 which had been digested with Banll and Fspl RENs. The products of this ligation reaction were transformed into E. coli strain HB101. Plasmid DNA from transformed colonies was purified and digested with Fokl. Clones which linearized with Fokl were sequenced. Plasmid pSY 1367 contained the desired MCS sequence and was chosen for subsequent constructions.

Plasmids pPT 0124 and pSY 1367 were digested with Nrul and Ncol and the DNA fragments were purif ied by agarose gel electrophoresis and NACS purification. The small fragment (approximately 500 bp) from pPT 0124 was ligated with the large fragment from pSY 1367. The product of the ligation mixture was transformed into E. coli. Plasmid DNA from single colonies was purified and analyzed by restriction digests and DNA sequencing. One plasmid, pPT 0134, contained the desired sequence and was used as the acceptor vector for the DCP constructions.

### SYNTHESIS AND ASSEMBLY OF THE C UNITS

Four oligonucleotide strands were synthesized and purified as described in Example 1. Each pair of oligonucleotide strands encodes a C₂ unit, either the 5' or 3' C₂ unit.

2.A) 5' - GTGCTCACGGCCCAGCAGGTCCGAAGGGCGCGCATGGCCCAGCAGGCCCGAAA

2.B) 3' -     AGTGCCGGGTCGTCCAGGCTTCCCGCGCGTACCGGGTCGTCCGGGCTTT

GGTGCC

CCACGGCACG

2.C) 5' - GTGCCCATGGCCCAGCAGGACCGAAAGGAGCTCACGGTCCGGCAGGTCCGAAAG

2.D) 3' - GTACCGGGTCGTCCTGGCTTTCCTCGAGTGCCAGGCCGTCCAGGCTTTCCACG

Oligonucleotide strands 2.A and 2.B were annealed and ligated with the DNA of plasmid pPT 0134 which had been digested with Fokl REN. The products of this ligation reaction were transformed into E. coli strain HB101. Plasmid DNA from transformed colonies was purified and digested with Sfil. Clones which linearized with Sfil were sequenced. Plasmid pPT 0135 contained the desired C₂ sequence and was chosen for subsequent constructions.

Strands 2.C and 2.D were annealed, ligated, and transformed as were strands 2.A and 2.B. Plasmid DNA from transformed colonies was purified and digested with Banll REN. Clones which linearized with Banll were sequenced. Plasmids pPT 0137 and pPT 0138 contained the correct C₂ DNA sequence.

Plasmid pPT 0135 and pPT 0137 were digested with Banl and Stul RENs. The large fragment from pPT 0135, containing the C₂ (strands A+B), was ligated with the small fragment of pPT 0137 containing the C₂ fragment (strands C+D). The ligation products were transformed into E. coli strain HB101. Plasmid DNA from transformants was purified and two digestions were performed, Sfil-Stul and Banll-Stul RENs, respectively. Clones that released a DNA fragment of approximately 800 bp in both digestions were sequenced. Plasmid pPT 0140 contained the correct C₂C₂ sequence, as shown in Table 2, and was used for DCP gene monomer constructions.

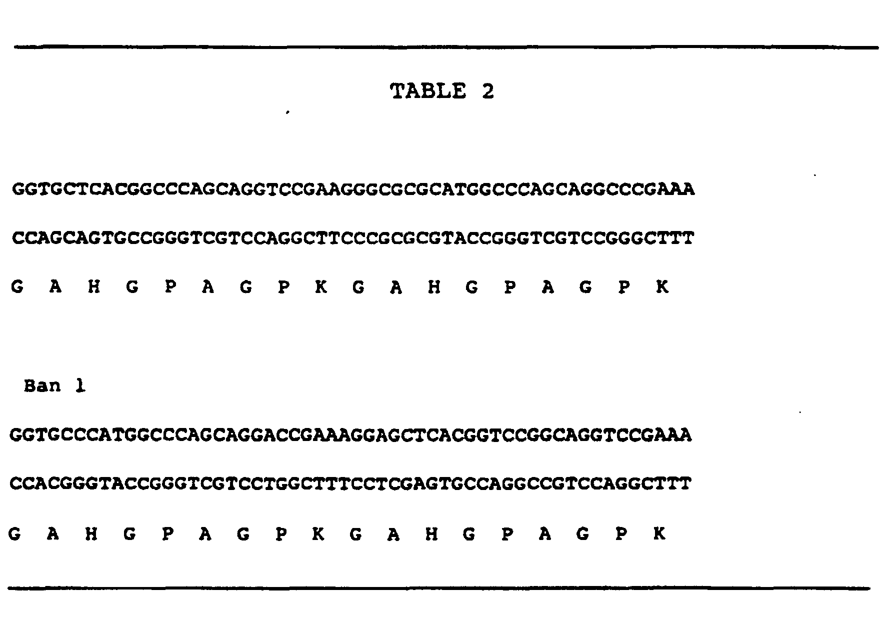

### DCP 1 and 2 GENE MONOMER CONSTRUCTIONS

Two oligonucleotide strands were synthesized and purified as described in Example 1.

3.A) 5′ - GTGCGCCTGGACCGGCTGGTCCACCGGGTGCTCCGGGACCTGCAGGCCCGCCAG

3.B) 3' - CGGACCTGGCCGACCAGGTGGCCCACGAGGCCCTGGACGTCCGGGCGGTCCACG

The two oligonucleotide strands encoding A₂(3A & 3B) were annealed and ligated with plasmid DNA pPT 0134 previously digested with Fokl REN. The products of the ligation mixture were transformed into E. coli strain HB101. Plasmid DNA from transformants was digested with Pstl REN and clones that were linearized were sequenced. Plasmid pPT 0142 was found to be correct and used for the multimerization of A₂ units.

Plasmid DNA from pPT 0142 was digested with Fokl REN and the fragment containing the A₂ unit was isolated by agarose gel electrophoresis and purified using a NACS column (see Example 1). The DNA fragment was self-ligated and the products of the ligation were ligated with pPT 0134 previously digested with Fokl REN. The products of the ligation mixture were transformed into E. coli strain HB101. Plasmid DNA from transformants was purified and digested with Fokl REN. Clones containing DNA inserts of 162 bp corresponding to A₆ were selected for sequence analysis. Plasmid pPT 0144 had the expected DNA sequence and was selected for subsequent constructions. Plasmid DNA from pPT 0144 was digested with Fokl REN and the two DNA fragments generated by the digestion were separated using agarose gel electrophoresis. The smaller DNA fragment was purified using a MACS column (see Example 1). The DNA fragment carrying the A₆ coding sequence was ligated with plasmid DNA pPT 0140 previously digested with Fokl. The products of the ligation were transformed into E. coli strain HB101. Plasmid DNA from individual colonies was analyzed for inserts containing multiple A₆ DNA fragments by digestion with Fokl. Several size inserts were found ranging from A₆ to A₂₄. One clone, pPT 0147 (shown in Table 3) was identified to contain the desired DCP 2 gene monomer sequence C₂A₁₂C₂ and was used for further constructions.

The clone containing the DCP1 gene monomer sequence C₂A₂₄C₂ (shown in Table 4), necessary to construct the DCP1 polymer gene, was found to be highly unstable during subsequent passages. This instability was attributed to the high copy number of the acceptor plasmid. For this reason, the gene monomer from this plasmid was recloned into pBR 322 (F. Bolivar, et al. (1977) Gene 2:95-113). The plasmid DNA containing the C₂A₂₄C₂ gene fragment was isolated from the plasmid by digestion with Nrul and EcoRV RENs, agarose gel electrophoresis, and purified on a NACS column. This DNA fragment was ligated with plasmid pBR322 DNA digested with EcoRV and Nrul RENs (see agarose ligation in Example 1). The products of this ligation were transformed into E. coli strain HB101 and selected on bacterial plates containing the antibiotic ampicillin at 100 µg/ml. Plasmid DNA from individual colonies was analyzed by agarose gel electrophoresis. Clones containing the insert were further analyzed by digestion with several RENs and one, pPT 0153, was chosen for the construction of the DCP 1 polymer gene. This plasmid was stable.

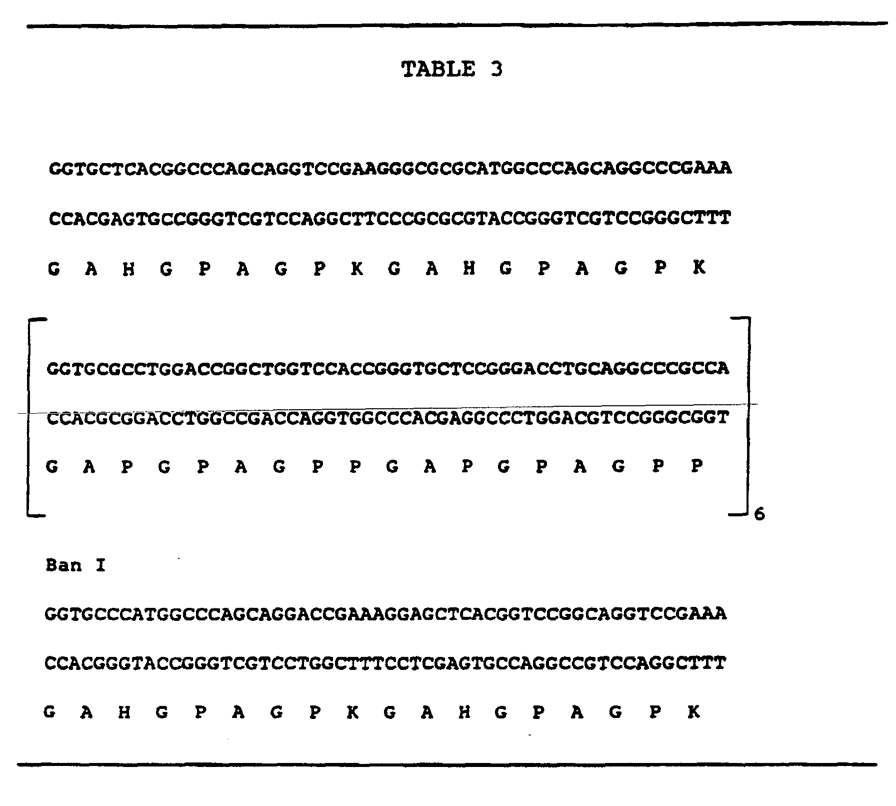

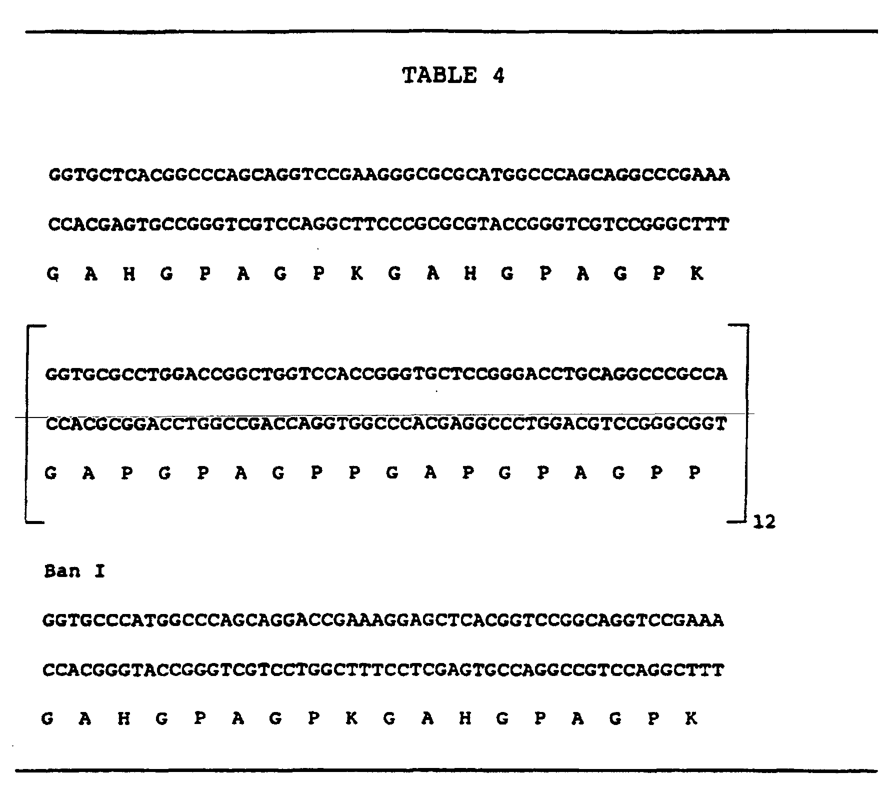

### DCP 1 POLYMER GENE CONSTRUCTION.

Plasmid from DNA pPT 0153 was digested with Acyl REN and subsequently with Fokl REN. The DNA fragment containing the DCP 1 gene monomer, 783 bp, was isolated by agarose gel electrophoresis and purified using a NACS column (see Example 1). The monomer gene fragment was ligated with pSY 1262 (see patent application number PCT/US87/02822) which had been digested with Banl REN. The ligation product was transformed into E. coli strain HB101 and selected for growth on bacterial plates containing the antibiotic kanamycin. Plasmid DNA from individual colonies was analyzed for inserts containing multiple DCP 1 monomer fragments by digestion with BamHl and Pvull RENs and electrophoresis on agarose gel. One clone, pPT 0164, contained the gene monomer (783 bp); another, pPT 0165, a slightly larger fragment (approximately 1200 bp); and a third, pPT 0166, a gene dimer (1566 bp).

### DCP 1 PROTEIN EXPRESSION ANALYSIS.

E. coli strain HB101 containing plasmid pPT 0164, pPT 0165 or pPT 0166 was grown at 30°C to an OD₆₀₀ of 0.7 and then shifted to 42°C for 2.0 hours. The proteins produced by these cells were analyzed by western blot analysis for novel protein bands which reacted with DCP-C₂A₂ peptide antisera. A band with an apparent molecular weight of approximately 45 kD was observed in the culture containing the plasmid pPT 0164. A band of 68 kD was observed with pPT 0165 and a light smear of bands with pPT 0166.

Because of the low level of detectable expression in the strain containing pPT 0166, the mRNA produced by the DCP 1 clones was analyzed. mRNA was prepared as described in Example 1. By northern blot analysis (see Example 1), the DCP specific mRNA from all clones, using as a probe the DCP2 monomer sequence, was shown to be full length and synthesized at approximately the same level regardless of the DCP gene size.

### DCP 1 pPT 0166 561 Amino Acids MW: 46,409 Dalton

MDPVVLQRRDWENPGVTQLNRLAAHPPFASDPM

[(GAHGPAGPK)2 (GAPGPAGPP)24 (GAHGPAGPK)2]2

GAMDPGRYQLSAGRYHYQLVWCQK

### DCP 2 POLYMER GENE CONSTRUCTION.

Plasmid DNA from pPT 0147 was digested with Fokl REN and the digestion fragments were separated by agarose gel electrophoresis. The DCP 2 gene fragment of 483 bp was excised and purified by NACS column (see Example 1). The purified fragment was ligated with pSY 1262 which had been digested with Banl REN. The products of this ligation were transformed into E. coli strain HB101 and the transformants were selected for growth on bacterial plates containing the antibiotic kanamycin. Plasmid DNA from individual colonies was purified and analyzed for multiple insertions of the DCP 2 gene monomer fragment. Several clones were obtained ranging in size from 500 to 3,000 bp. See Table 5 for the results.

### DCP 2 PROTEIN EXPRESSION ANALYSIS

E. coli strain HB101 containing plasmids pPT 0155 to 0163 were grown at 30°C to an OD₆₀₀ of 0.7 and then shifted to 42°C for 2.0 hours. The proteins produced by these cells were analyzed by western blot analysis for a protein band reactive with DCP-C₂A₂ peptide specific antisera. See Table 5 for the results.

**TABLE 5**

| DCP 2 Expression clones | # of repeats | Gene size (in bp) | # of amino acids | Protein band observed (in kD) |
|---|---|---|---|---|
| pPT 155 | 1 | 603 | 201 | no detection |
| pPT 156 | 2 | 1035 | 345 | 40 |
| pPT 157 | 3 | 1467 | 489 | 60 |
| pPT 158 | 4 | 1899 | 633 | 80 |
| pPT 159 | 5 | 2331 | 777 | 100 |
| pPT 160 | 6 | 2763 | 921 | smear |
| pPT 161 | 7 | 3195 | 1065 | no detection |
| pPT 162 | 7+ | 3240 | 1080 | no detection |
| pPT 163 | 7+ | 3420 | 1140 | no detection |

### DCP 2 pPT 0159 777 Amino Acids MW: 64,094 Dalton

MDPVVLQRRDWENPGVTQLNRLAAHPPFASDPM

[(GAHGPAGPK)2 (GAPGPAGPP)12 (GAHGPAGPK)2]5

GAMDPGRYQLSAGRYHYQLVWCQK

### DCP 3 MONOMER CONSTRUCTION

Four oligonucleotide strands encoding (AB)₂ were synthesized and purified as described (see Example 1).

4.A) 5' - GTGCTCCGGGACCTGCAGAATATTATTCTAGAGGTGACCCAGGACCGCCTG - 3'

4.B) 3' - AGGCCCTGGACGTCTTATAATAAGATCTCCACTGGGTCCTGGCGCACCACG 5'

4.C) 5' - GGCCCACCGGGTAGCCGTGGCGATCCGGGACCACCGG-

4.D) 3' - ACGTCCGGGTGGCCCATCGGCACCGCTAGGCCCTGGTGGCC-

GTGCACCTGGCCCAGCGGGTCCGCCTGGAT - 3'

CACGTGGACCGGGTCGCCCAGGCGGACCTAGATC - 5'

Oligonucleotide strands 4.A and 4.B were annealed and ligated with the DNA plasmid pPT 0134 (see Example 1) which had been digested with Fokl REN. The products of this ligation were transformed into E. coli strain HB101. Plasmid DNA from transformed colonies was purified and digested with Pstl and Stul. Clones containing a fragment of approximately 800 bp were sequenced. Plasmid pPT 0139, containing the desired sequence of strands 4.A and 4.B, was chosen for subsequent constructions.

Strands 4.C and 4.D were annealed and ligated with the DNA plasmid pPT 0139 which had been previously digested with Xbal and Pstl RENs. The products of this ligation were transformed into E. coli strain HB101. Plasmid DNA from transformed colonies was purified and digested with Ncol and Dralll RENs. Clones containing a DNA fragment corresponding to the combined insertion of strands 4.A and B and 4.C and D were sequenced. Plasmid pPT 0143, containing the correct (AB)₂ DNA sequence, as shown in Table 6, was chosen for further constructions.

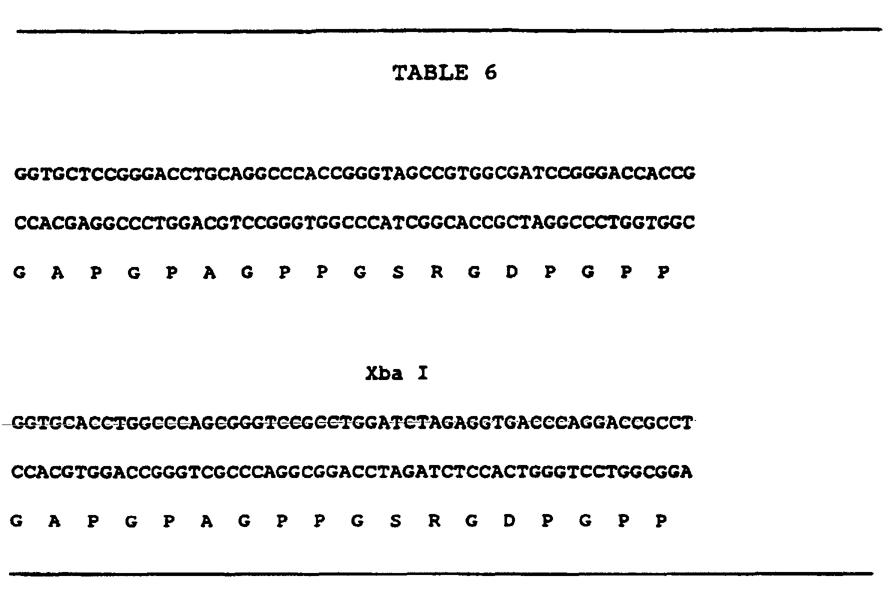

Plasmid DNA from pPT 0143 was digested with Fokl REN and the fragment containing the (AB)₂ gene fragment was isolated by agarose gel electrophoresis and purified on a NACS column. The DNA fragment was then ligated with pPT 0134 that had been digested with Fokl REN. The products of the ligation were transformed into E. coli strain HB101 and selected for growth on bacterial plates containing the antibiotic chloramphenicol. Plasmid DNA from individual colonies was analyzed for inserts containing multiple (AB)₂ DNA fragments by digestion with Fokl REN. Several clones were obtained ranging from one copy to several copies of (AB)₂. One clone, pPT 0169, containing (AB)₆ was used as an intermediate for the construction of the DCP 3 gene monomer.

The (AB)₆ gene fragment was purified from pPT 0169 by digestion with Fokl REN, agarose gel electrophoresis and NACS purification. This DNA fragment was then ligated with DNA plasmid pPT 0140 that had been previously digested with Banl REN. The products of the ligation were transformed into E. coli strain HB101, and transformants were selected on bacterial plates containing the antibiotic chloramphenicol. Plasmid DNA from individual colonies was analyzed by digestion with Fokl REN for inserts containing multiple copies of the (AB)₆ gene fragment. One clone, pPT 0171, containing C₂(AB)₁₂C₂ (shown in Table 7) was chosen as the DCP 3 gene monomer for subsequent constructions.

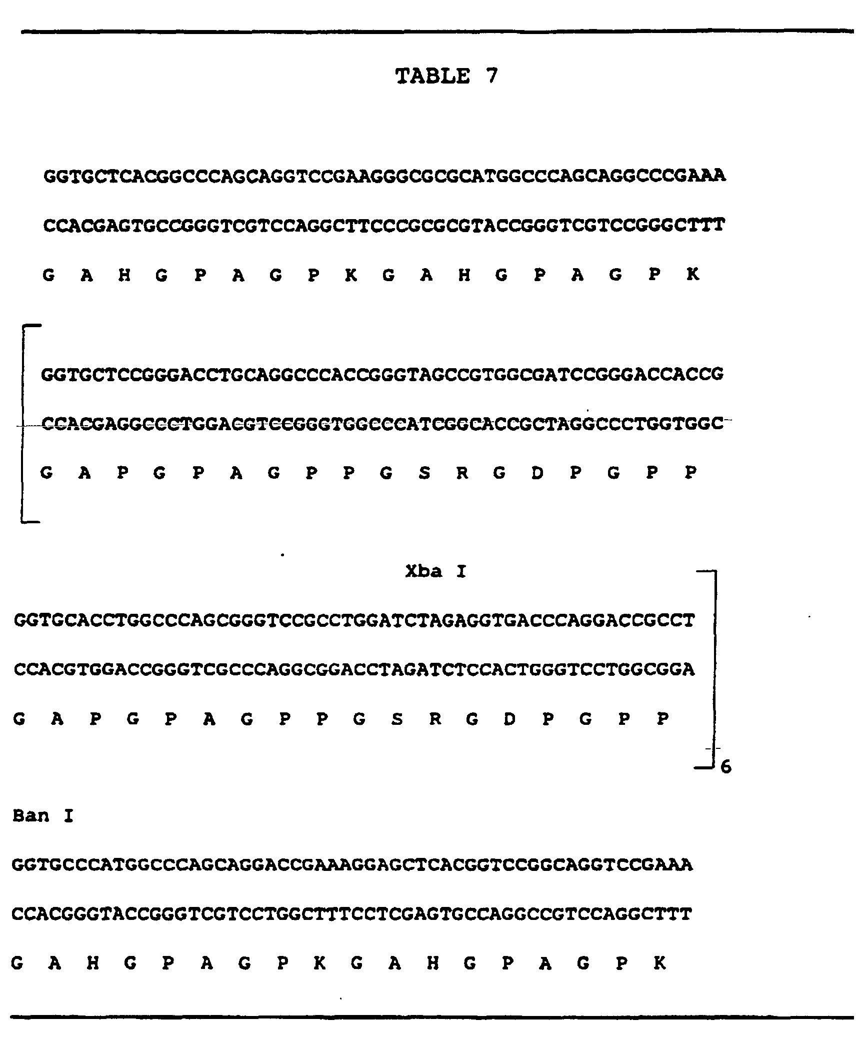

### DCP 3 POLYMER GENE CONSTRUCTION

Plasmid DNA from pPT 0171 was digested with Fokl REN and the fragment containing the DCP 3 gene monomer purified by agarose gel electrophoresis and NACS purification. The DCP 3 monomer was then self-ligated and then ligated with DNA plasmid pSY 1262 that had been digested with Banl REN. The products of the ligation were transformed into E. coli strain HB101 and selected for growth on bacterial plates containing the antibiotic kanamycin. Plasmid DNA from individual colonies was purified and analyzed after digestion with Xcml and Pvull RENs for insertions containing multiple copies of the DCP 3 gene monomer fragment. Clones pPT 0173, pPT 0174, pPT 0175 and pPT 0176 containing monomer, dimer, trimer and tetramer forms of DCP 3, respectively, were selected for expression analysis.

### DCP 3 PROTEIN EXPRESSION ANALYSIS.

E. coli strain HB101 containing DCP 3 plasmids pPT 0173, pPT 0174, pPT 0175, or pPT 0176 were grown at 30°C to an OD₆₀₀ of 0.7 and then shifted to 42°C for 2.0 hours. The proteins produced by these cells were analyzed by western blot analysis for novel protein bands reactive with DCP-(AB)₂ peptide specific antisera. Reactive bands were observed with each clone (see Table 8 for results). However, the expression of the full length polymer decreased with the increased size of the genes. Northern analysis using the DCP3 monomer as a probe showed that the synthesis of full length mRNA in these clones was at equivalent levels.

**TABLE 8**

| DCP 3 Expression clones | # of repeats | Gene size (in bp) | # of amino acids | Protein band observed (in kD) |
|---|---|---|---|---|
| pPT 0173 | 1 | 927 | 309 | 28 |
| pPT 0174 | 2 | 1683 | 561 | 64 |
| pPT 0175 | 3 | 2439 | 813 | 98 |
| pPT 0176 | 4 | 3195 | 1065 | 135 |

### DCP 3 pPT 0176 1065 Amino Acids MW: 91,966 Dalton

MDPVVLQRRDWENPGVTQLNRLAAHPPFASDPM

{(GAHGPAGPK)2 [(GAPGPAGPP)(GSRGDPGPP)]12 (GAHGPAGPK)2}4

GAMDPGRYQLSAGRYHQLVWCQK

### Example 3

**TABLE 9**

| STRUCTURE DCP 4, 5, & 6 | |
|---|---|
| DCP 4 | [C₂(DB)₁₂C₂]₄ |
| DCP 5 | [C₂(DB)₆C₂]₄ |
| DCP 6 | [C₂D₂₄C₂]₄ |

where:
B=GSRGDPGPP
C=GAHGPAGPK
D=GAQGPAGPG

### DCP 4 AND 5 GENE MONOMER CONSTRUCTIONS

Three double stranded DNA sections, coding for (DB)₃, were synthesized, purified and annealed as described in Example 1.

Eco0109I

5.A) 5' - GTGCACAGGGACCGGCGGACCAGGTGGCTCTCGAGGCGATCCGGGTCCTCCGG

5.B) 3' - TGTCCCTGGCCGCCCTGGTCCACCGAGAGCTCCGCTAGGCCCAGGAGGCCCACG

Eco0109I Dra111

5.C) 5'- GTGCACAAGGACCGGCAGGCCCTGGTGGCAGCCGCGGTGATCCGGGCCCACCGG

5.D) 3'- TGTTCCTGGCCGTCCGGGACCACCGTCGGCGCCACTAGGCCCGGGTGGCCCACG

5.E) 5'- GTGCTCAAGGACCGGCTGGCCCAGGCGGTTCCCGTGGAGACCCGGGTCCACCGG

5.F) 3'- AGTTCCTGGCCGACCGGGTCCGCCAAGGGCACCTCTGGGCCCAGGTGGCCCACG

The three DNA sections were cloned separately. The first two strand section (5.A & 5.B) was ligated to pPT 0138 previously digested with Banl REN. The ligation products were transformed into E. coli strain HB101 and selected for growth on bacterial plates containing the antibiotic chloramphenicol. Plasmid DNA from individual colonies was digested with Eco0109I and Stul RENs and analyzed by agarose gel electrophoresis. Plasmid DNA containing an appropriately sized DNA fragment was sequenced and one clone, pPT 0221, was used in subsequent constructions.

The second pair of oligonucleotide strands (5.C & 5.D) were ligated with pPT 0134 which had been digested with Fokl REN. After transformation of E. coli, plasmid DNA from individual colonies was analyzed by digestion with Banll and Stul RENs . DNAs that were digested by both enzymes were sequenced. One clone, pPT 0222, had the expected sequence and was used for subsequent constructions.

Plasmid DNA from pPT 0222 was digested with Fokl REN and the fragment (54 bp) containing the second pair of oligonucleotide strands (5C & 5D) was isolated by agarose gel electrophoresis followed by NACS purification. This DNA fragment was ligated with pPT 0221 previously digested with Banl REN. The products of the ligation were transformed into E. coli and plasmid DNA from single colonies was analyzed by agarose gel electrophoresis after digestion with Dralll and Stul RENs. One clone, pPT 0223, was chosen, after DNA sequencing, to be the acceptor vector for the third synthesized DCP gene fragment.

Plasmid pPT 0223 was digested with Banl REN and ligated with the third pair of oligonucleotides (5.E & 5.F). The product of the ligation reactions was transformed into E. coli strain HB101 and selected for growth on bacterial plates containing the antibiotic chloramphenicol. Plasmid DNA from individual transformants was purified and digested with Fokl and Banl RENs. Clones containing the correct fragment size were sequenced. One clone, pPT0224, shown in Table 10, containing the desired sequence was used in the subsequent constructions of the DCP 4 and DCP 5 monomers.

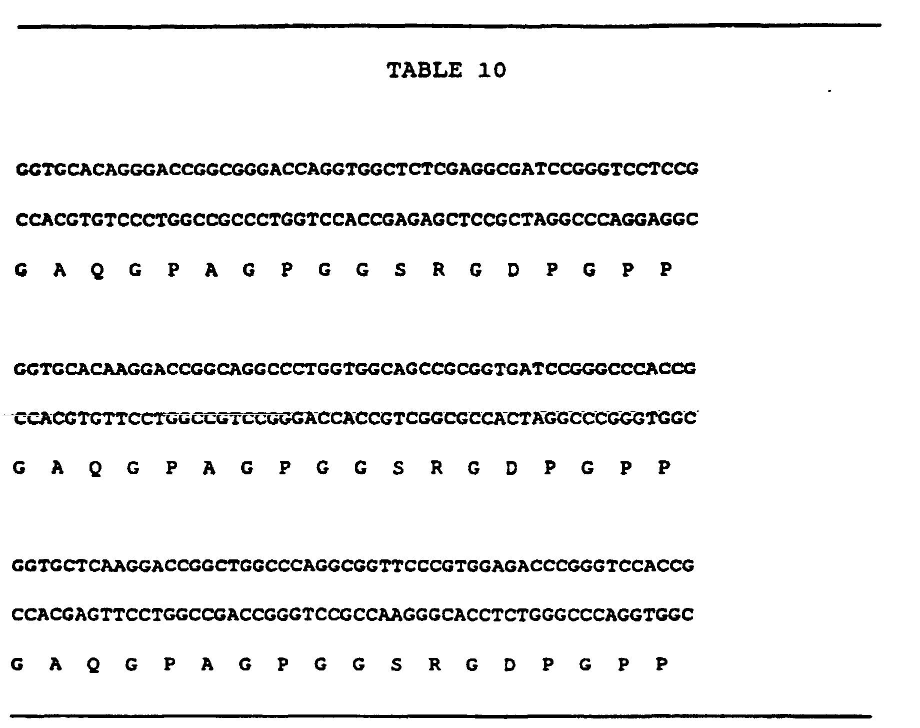

Plasmid DNA from pPT 0224 was digested with Fokl and Banl RENs and the digestion fragment carrying (DB)₃ was isolated by agarose gel electrophoresis and purified on a NACS column. The purified fragment was self-ligated and then cloned into pPT 0140 which had been digested with Banl REN. The products of the ligation were transformed into E. coli strain HB 101. Plasmid DNA from individual colonies was analyzed for inserts containing multiple (DB)₃ DNA fragments by digestion with Fokl. Several size inserts were found ranging from (DB)₃ to (DB)₁₂. One clone, pPT 0229, was identified to contain the desired DCP 5 monomer sequence, C₂(DB)₆C₂, and was used for subsequent constructions. The clone containing the DCP 4 gene monomer sequence C₂(DB)₁₂C₂ was found to be highly unstable during subsequent passages, as was observed during the construction of DCP 1. Subsequently, a new plasmid was constructed to use as an acceptor for the DCP 4 gene monomer fragment.

Plasmid pACYC 184 (Chang and Cohen, *J. Bacteriol.* 134, 1141-1156 [1977]) was digested with BanI REN, purified by agarose gel electrophoresis, and the DNA fragment corresponding to approximately 2000 bp was further purified using a NACS column. This DNA fragment was filled in using DNA polymerase (see Example 1) and then self-ligated. The products of the ligation mixture were transformed into E. coli strain HB101 and selected on bacterial plates containing chloramphenicol at 30 µg/ml. Plasmid DNA from individual colonies was linearized by digestion with Eco47III. One clone, pPT 0235, was used as the acceptor vector for the DCP 4 monomer.

The plasmid DNA containing the DCP 4 gene monomer fragment was isolated from the plasmid by digestion with DraI and PvuII RENs, followed by agarose gel electrophoresis. The agarose slice containing the DCP 4 gene monomer fragment was ligated with plasmid pPT 0235 previously digested with Eco47III REN, and purified by agarose gel electrophoresis and NACS column. The products of this ligation were transformed into E. coli strain HB101 and selected on bacterial plates containing chloramphenicol at 30 µg/ml. Plasmid DNA from individual colonies was analyzed by digestion with NruI and EcoRV RENs. One clone containing the DCP 4 gene monomer fragment, pPT 0237, was chosen for the construction of the DCP 4 polymer gene. This plasmid was stably maintained in E. coli.

### DCP 4 POLYMER GENE CONSTRUCTION

Plasmid DNA from pPT 0237 was digested with Fokl REN. The DNA fragment containing the DCP 4 gene monomer was isolated by agarose gel electrophoresis and purified using a NACS column (see Example 1). The monomer gene fragment was then ligated with pSY 1262 which had been previously digested with Banl REN, treated with phosphatase, and purified by gel electrophoresis and MACS column. The ligation products were transformed into E. coli strain HB101 and the transformants were selected for growth on bacterial plates containing the antibiotic kanamycin at 50 µg/ml. Plasmid DNA from individual colonies was purified and analyzed for multiple insertion of the DCP 4 gene monomer fragment. Several clones were obtained containing one, two or four copies of the DCP 4 gene monomer. Plasmids pPT0247, pPT0248, and pPT0249 containing respectively 1, 2, and 4 copies of the gene monomer, were selected for protein expression analysis.

### DCP 4 PROTEIN EXPRESSION ANALYSIS

E. coli strain HB101 containing plasmids pPT 0247, pPT 0248, and pPT 0249 were grown at 30°C to an OD₆₀₀ of 0.7 and then shifted to 42°C for 2.0 hours. The proteins produced by these cells were analyzed by western blot analysis for novel protein bands reactive with DCP-C₂(A₂) peptide specific antisera. A reactive band corresponding to full length product was observed with each clone.

### DCP 4 pPT 0249 1065 Amino Acids MW: 91,533 Dalton

MDPVVLQRRDWENPGVTQLNRLAAHPPFASDPM

{(GAHGPAGPK)2 [(GAQGPAGPG)(GSRGDPGPP)]12 (GAHGPAGPK)2}4

GAMDPGRYQLSAGRYHYQLVWCCK

### DCP 5 POLYMER GENE CONSTRUCTION

Plasmid DNA from pPT 0229 containing the DCP 5 gene monomer was isolated after digestion with Fokl REN by agarose gel electrophoresis followed by NACS purification. The gene fragment was ligated with pSY 1262 which had been digested with Banl REN. The products of the ligation were transformed into E. coli strain HB101 and selected for growth on bacterial plates containing the antibiotic kanamycin at 50 µg/ml. Plasmid DNA from individual colonies was purified and analyzed for inserts containing multiple copies of the DCP 5 gene monomer sequence C₂(DB)₆C₂. Plasmids pPT 0231 and pPT 0232, respectively, contained three and four copies of the DCP 5 gene monomer and were used for expression analysis.

### DCP 5 PROTEIN EXPRESSION ANALYSIS

E. coli strain HB101 containing plasmids pPT 0231 and pPT 0232 were grown at 30°C to an OD₆₀₀ of 0.7 and then shifted to 42°C for 2.0 hours. The proteins produced by these cells were analyzed by western blot analysis for novel protein bands reactive with DCP-C₂A₂ peptide specific antisera. A reactive band corresponding to full length product was observed with each clone.

### DCP 5 pPT0232 633 Amino Acids MW: 55,228 Daltons

MDPVVLQRRDWENPGVTQLNRLAAHPPFASDPM

{(GAHGPAGPK)2 [ (GAQGPAGPG)(GSRGDPGPP)]6 (GAHGPAGPK)2 }4

GAMDPGRYQLSAGRYHYQLVWCQK

### DCP 6 GENE MONOMER CONSTRUCTION

Four oligonucleotide strands encoding D₄, were synthesized, purified and annealed as described in Example 1.

Eco0109I

6.A) 5' - GTGCACAGGGACCGGCGGGTCCAGGCGGTGCTCAAGGACCGGCAGGCCCTTAATTAAG

6.B) 3' - TGTCCCTGGCCGCCCAGGTCCGCCACGAGTTCCTGGCCGTCCGGGAATTAATT-
CCACG

(EcoO109I)

6.C) 5′ - GCCCTGGTGGCGCTCAAGGTCCGGCTGGCCCAGGAGGCGCGCAGGGTCCGGCAGGT-

6.D) 3' -    GACCACCGCGAGTTCCAGGCCGACCGGGTCCTCCGCGTCCCAGGCCGTCCA-

CCGGGAG

GGCCCTCCACG

The first oligonucleotide segment consisting of strands 6A and 6B was ligated to pPT 0138 previously digested with Banl REN. The ligation products were transformed into E. coli strain HB101 and selected for growth on bacterial plates containing the antibiotic chloramphenicol. Plasmid DNA from individual colonies was digested with Eco0109I and Xmnl RENs and analyzed by agarose gel electrophoresis. Plasmid DNA containing the correct sized fragment was sequenced and one clone, pPT 0219, containing the correct sequence was used for subsequent constructions.

Plasmid pPT 0219 was digested with Banl and Eco0109I RENs and ligated with the second pair of oligonucleotides (6C and 6D). The products of the ligation were transformed into E. coli strain HB101 and selected for growth on bacterial plates containing the antibiotic chloramphenicol. Plasmid DNA from transformants was purified and digested with Fokl and Banl RENs. Clones containing the correct fragment size were sequenced. One clone, pPT 0220, containing the sequence shown in Table 11 was used in subsequent constructions of the DCP 6 gene monomer.

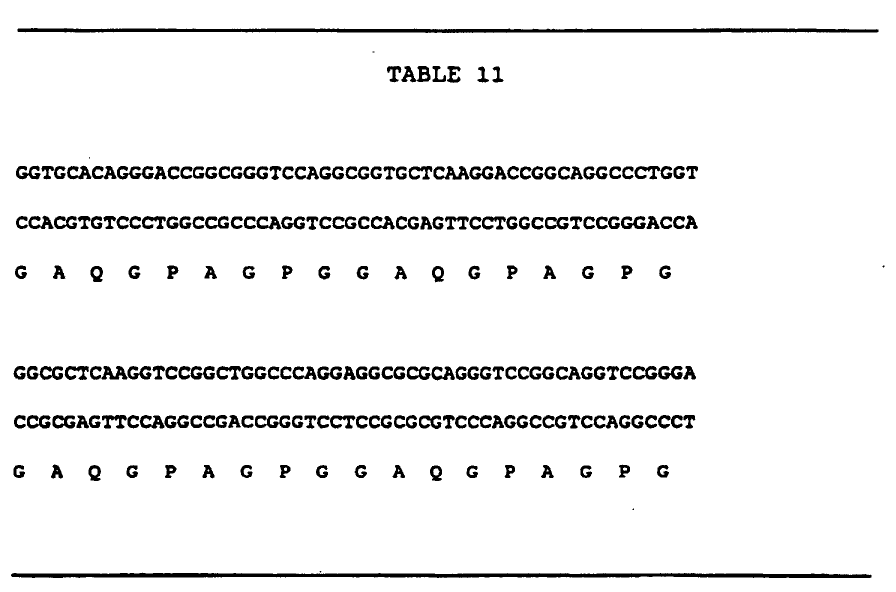

Plasmid DNA containing the D₄ sequence was digested with Fokl and Banl RENs and the digestion fragment containing D₄ was isolated by agarose gel electrophoresis followed by MACS purification. The purified fragment was self-ligated and subsequently ligated with DNA plasmid pPT 0140 digested with Banl REN. The ligation products were transformed into E. coli strain HB101 and selected for growth on bacterial plates containing the antibiotic chloramphenicol. A transformant, pPT 0242, containing the DCP 6 gene monomer ((D₄)₆ flanked by C₂-C₂) was used for subsequent constructions.

### DCP 6 POLYMER GENE CONSTRUCTION

Plasmid DNA from pPT 0242 was digested with Fokl REN. The DNA fragment containing the DCP 6 gene monomer was isolated by agarose gel electrophoresis and purified using a NACS column. The monomer gene fragment was then ligated with pSY 1262 which had been previously digested with Banl REN, treated with phosphatase, and purified by gel electrophoresis and NACS column. The ligation products were transformed into E. coli strain HB101 and the transformants were selected for growth on bacterial plates containing the antibiotic kanamycin at 50 µg/ml. Plasmid DNA from individual colonies was purified and analyzed for multiple insertion of the DCP 6 gene monomer sequence, C₂D₂₄C₂. Several clones were obtained ranging from one to four repeats of the DCP 6 gene monomer. Plasmids pPT 0243, pPT 0244, pPT 0245, and pPT 0246 containing respectively 1, 2, 3, and 4 repeats of the gene monomer were selected for protein expression analysis.

### DCP 6 PROTEIN EXPRESSION ANALYSIS

E. coli strain HB101 containing plasmids pPT 0243 to pPT 0246 were grown at 30°C to an OD₆₀₀ of 0.7 and then shifted to 42°C for 2.0 hours. The proteins produced by these cells were analyzed by western blot analysis for novel protein bands reactive with DCP-C₂A₂ peptide specific antisera. See Table 11 for the results.

**TABLE 11**

| DCP 6 Expression clones | # of repeats | Gene size (in bp) | # of amino acids | Protein band observed (in kD) |
|---|---|---|---|---|
| pPT0243 | 1 | 927 | 309 | no detection |
| pPT0244 | 2 | 1683 | 561 | 72 |
| pPT0245 | 3 | 2439 | 813 | 110 |
| pPT0246 | 4 | 3195 | 1065 | 140 |

### pPT0246 1,065 Amino Acids MW: 85,386 Dalton

MDPVVLQRRDWENPGVTQLNRLAAHPPFASDPM

[(GAHGPAGPK)2 (GAQGPAGPG)24 (GAHGPAGPK)2]4

GAMDPGRYQLSAGRYHYQLVWCCK

### PROPERTIES OF PURIFIED DCP6

The protein polymer, DCP6, was purified in multigram quantities as produced from E. coli strain pPT 0246 using standard protein purification, extraction, and separation methods. The lyophilized product was a white, spongy material. By amino acid compositional analysis, the product was shown to consist primarily of the amino acids glycine, alanine, proline and glutamine. The molar % glycine+alanine+ proline out of the total amino acids were 82.8%. The molar ratio of glycine, alanine, proline, and glutamine was 1.95:0.89:1.00:0.89, respectively. The theoretical ratio of these amino acids for DCP6 polymer is 1.90:1.00:1.00:0.43.

The purified product was dried and analyzed for elemental composition. The chemical analysis showed the product to contain 50.5% carbon, 6.78% hydrogen, 19.5% nitrogen, 11.3% water and less than 0.1% noncombustible ash. Theoretical elemental composition of DCP6 is 50.9% carbon, 6.49% hydrogen and 20.2% nitrogen. These analyses indicate that the dried product consists of approximately 85.7% protein, and that approximately 98.6% of that protein is DCP6.

The dried product is extremely soluble in water. 8% weight solutions or greater can be easily produced. At room temperature or above such solutions are viscous but fluid. Upon chilling to 0°C the solution forms a solid gel which does not flow and is semi-transparent. Upon heating to greater than 28°C, the gel forms a thick solution. This therm moreversible transition between the liguid and gel phases of the polymer solution can occur repeatedly with no apparent physical change in the polymer structure.

### PLASMID pPT 0285 CONSTRUCTION

Two oligonucleotide strands were synthesized and purified as described in Example 1: 
(Eco47III)PmeI      PmlI      NruI      BanI
StuI EcoRV SnaBI(SnaI)
1.    5'-    GCTATGTTTAAACCACGTGTTCGCGATCCGGGTGCCGATCCAGGCCTGCGATATCA-
2.    3'-    CGATACAAATTTGGTGCACAAGCGCTAGGCCCACGGCTAGGTCCGGACGCTATAGT-
             A  M  F  K  P  R  V  R  D  P  G  A  D  P  G  L  R  Y  Q

             (5' [continued])   GTACGTA
             (3' [continued])   CATGCAT
                                 Y  V
 The two oligonucleotide strands were annealed and ligated with the DNA of plasmid pPT 0235 which had been digested with Eco47III and SnaI RENs.

The product of this ligation reaction was transformed into E. coli strain HB101. Plasmid DNA from transformants was purified and digested with EcoRI in combination with Eco47III or SnaI or NruI RENs. Plasmid DNA from two clones that gave the correct digestion pattern was sequenced. One plasmid, designated pPT 0285, was found to be correct and chosen for further contstructions.

### CLP 3.1 GENE MONOMER CONSTRUCTION

The CLP 3.1 synthetic gene was assembled from smaller parts. First, four double-stranded sections of DNA were chemically synthesized.

FRAGMENT 1:

BanI                                                    PstI      BanII   BanI
5'- GTGCCCCTGGCGCTCCGGGTTCTCAAGGTGCACCGGGTCTGCAGAAAGGGCTCG
3'-      GGGACCGCGAGGCCCAAGAGTTCCACGTGGCCCAGACGTCTTTCCCGAGCCACG

FRAGMENT 2:
PstI BanII
5'- GGGTGCACCGGGAGCGCCAGGTAGCCAGGGTGCACCGGGATTGCAGGGGGCT
3'- ACGTCCCACGTGGCCCTCGCGGTCCATCGGTCCCACGTGGCCCTAACGTCCC

FRAGMENT 3:

BanII                                                    DraIII
5'-     CCGGGTGCACCAGGTAGCCAGGGAGCACCGGGTCTGCAAGGAGCACCGG
3'- CCGAGGCCCAGCTGGTCCATCGGTCCCTCGTCCGCCAGACGTTCCTCGTG

FRAGMENT 4:

DraIII BamHI BanI StuI EcoRV
5'- CAAACACCGGGTGCACCGGATCCCAGGGCGCTCCGGCCTGCAAGGTGCCAGGCCTCGAT
3'-CCGAGTTTGTGGCCCACGTGGCCTAGGGTCCCGCAGGCCGGACGTTCCACGGTCCGGAGCAT

The two strands of fragment 3 were phosphorylated before annealing using Polynucleotide Kinase 3'-phosphatase-free (see Example 1). All of the fragments were then individually annealed using the procedure described in Example 1.

Fragment 1, after annealing, was digested with PstI REN; the digestion mixture was treated with a BioSpin column as was the phosphorylated fragment 3 strands. All four fragments were then loaded on a 12% polyacrylamide gel in 0.5 x TBE and the bands corresponding to double stranded DNA were identified by UV shadowing, excised from the gel and extracted by the crush and soak procedure (Smith, Methods *in Enzymology,* 65, 371-379 [1980]).

The fragments were then ligated into pPT 0285 previously digested with BanI and EcoRV RENs and further purified by MACS column (see Example 1). The product of the ligation reaction was transformed into E. coli strain HB101. Plasmid DNA from transformants was purified and digested with PvuII and SacII; clones containing inserts of the correct size were further analyzed with EcoRI in combination with several restriction endonucleases to determine the presence of unique restriction endonuclease sites prior to sequencing. One clone looked promising and was sequenced. Plasmid pPT 0291 contained the sequence shown in Table 12, containing all four fragments, but had a deletion of 17 bases in fragment 3 including the DraIII restriction site.

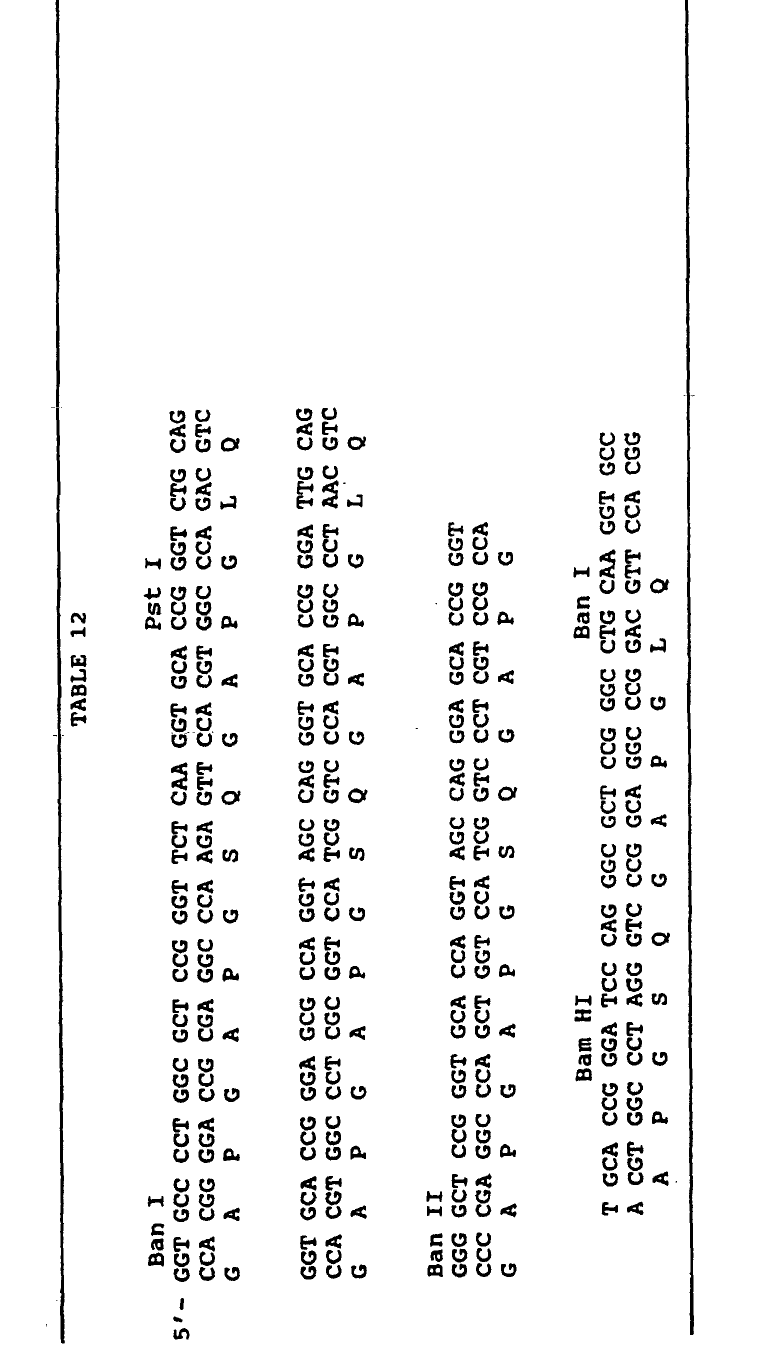

This plasmid was used to construct the CLP 3.1 monomer. Fragment 3 was ligated with fragment 4 using the HCC ligation method, see Example 1. The product of the ligation mixture was electrophoresed in 2% low melting agarose gel and the band corresponding to the ligated product was excised from the gel and ligated with pPT 0291 plasmid DNA that had been previously digested with BanII and SnaBI RENs and purified by agarose gel electrophoresis and NACS column.

The product of the ligation reaction was transformed into E. coli strain HB101. Plasmid DNA from transformants was purified and digested with BamHI and EcoRI RENs; clones that showed the correct restriction pattern were further analyzed with BanII or DraIII RENs; three clones containing inserts of the correct size were sequenced. Plasmid pPT 0292 contained the desired CLP 3.1 monomer sequence (see Table 13).

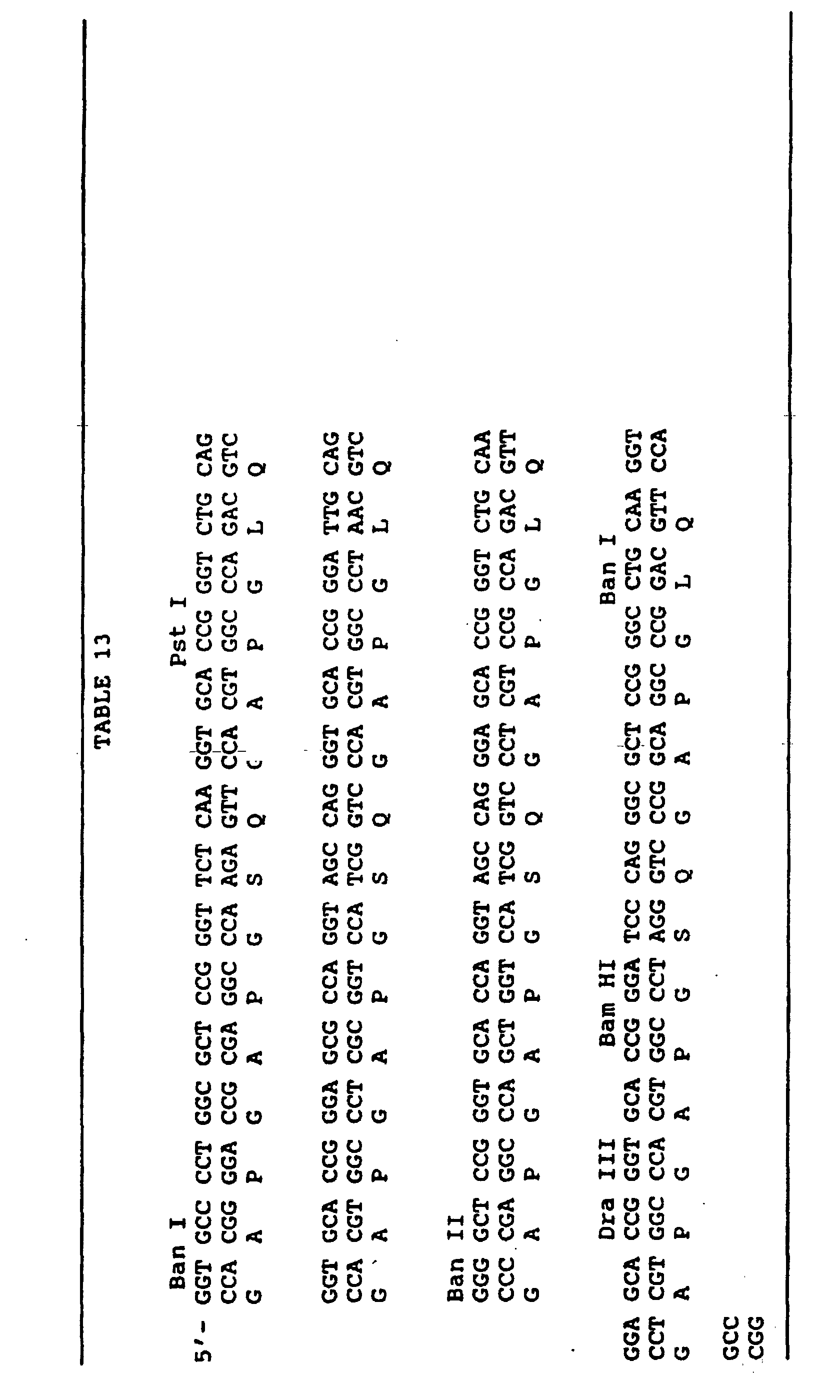

### CLP 3.1 POLYMER GENE CONSTRUCTION

Plasmid DNA pSY 1262 was digested with BanI REN. The digestion mixture was divided into two aliquots. One was treated with Calf Intestinal Phosphatase and the other was treated with Shrimp Alkaline Phosphatase (SAP), as described in Example 1.

Plasmid DNA from pPT 0292 was digested with BanI REN and the digestion fragments were separated by agarose gel electrophoresis. The CLP 3.1 gene fragment, 180 bp, was excised and purified by NACS column (see Example 1) and then ligated with plasmid pSY 1262 prepared as described above.

The product of this ligation reaction was transformed into E. coli strain HB101. Transformants were selected for resistance to kanamycin. Plasmid DNA from individual transformants was purified and analyzed for increase size due to CLP 3.1 multiple DNA insertion. Several clones were obtained ranging in size from approximately 1.0 kbp to 3.0 kbp. Five clones, pPT 0293 to pPT 0297, containing respectively 6, 9, 11, 13, and 17 repeats of the gene monomer were selected for protein expression analysis.

### CLP 3.1 PROTEIN EXPRESSION ANALYSIS

Overnight cultures of E. coli strain HB101 containing plasmids pPT 0293 to pPT 0297 which had been grown at 30°C were separately used to inoculate 50 ml of media contained in a 250 ml flask. Kanamycin was added at a final concentration of 50 µg per ml and the cultures were incubated with agitation (200 rpm) at 30°C. When the cultures reached an OD₆₀₀ of 0.8, 40 ml of each culture were transferred to a new flask prewarmed at 42°C and incubated at the same temperature for approximately 2 hours. The cultures (30° and 42°) were chilled on ice and OD₆₀₀ was taken. Cells were collected by centrifugation and divided into 1.0 OD₆₀₀ aliquots which were then used to perform dot blot and western blot analysis using CLP 3.1 peptide specific antisera (see Example 1). See Table 14 for the results. For purification and amino acids analysis larger cultures were used.

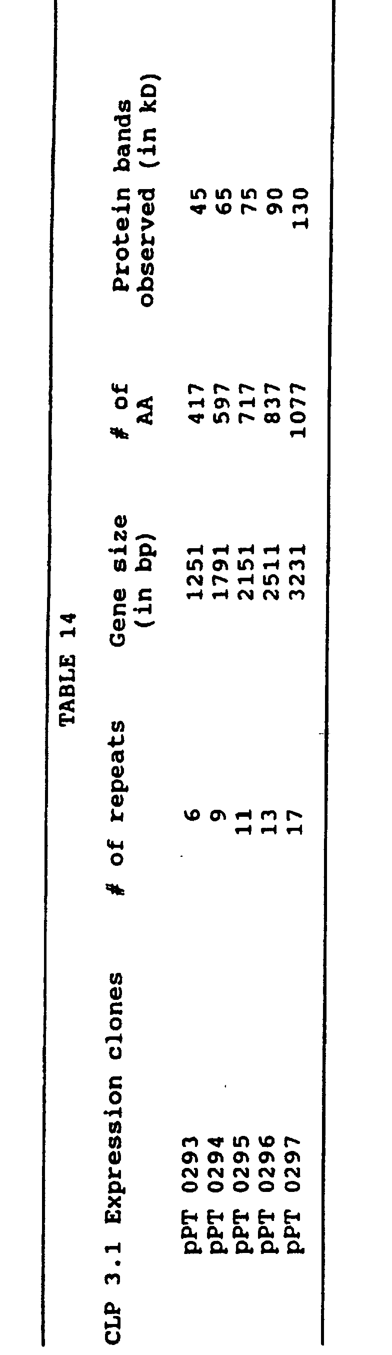

E. coli strain HB101 containing plasmids pPT 0293 to pPT 0297 were grown as described above. The proteins produced by these cells were analyzed by SDS-PAGE for detection of reactivity to CLP 3.1 peptide specific antisera. In every analysis a strong reactive band was observed of an apparent molecular weight from 45 kD to 130 kD respectively.

pPT 0297 1077 Amino acids MW: 91,266 Daltons

MDPVVLQRRDWENPGVTQLNRLAAHPPFASDPM

(GAPGAPGSQGAPGLQ)68

GAMDPGRYQLSAGRYHYQLWVCQK

As evidenced by the above results, high molecular weight collagen-like polymers can be produced. The polymers are characterized by having repeating triads with the first amino acid of each triad being glycine. By lowering the percentage of prolines as compared to natural collagen, a variety of useful collagen-like polymers are formed having properties similar to collagen, but enjoying unique characteristics. The products find use as films, fibers, molded objects, admixed with other natural or synthetic polymers or coatings on fibers, films, labware or other surfaces, e.g., prosthetic devices, and the like.

All publications and patent applications mentioned in this specification are indicative of the level of skill of those skilled in the art to which this invention pertains.

Although the foregoing invention has been described in some detail by way of illustration and example for purposes of clarity of understanding, it will be understood that certain changes and modifications may be practiced within the scope of the appended claims.

## Claims

1. A collagen-like polymer of at least 30 kD as determined by SDS-PAGE, obtainable by expression in a unicellular organism from a construct prepared in vitro,
comprising at least 60 weight % of triads having glycine as the first amino acid and at least 40 number % said triads comprising at least one proline wherein the overall proline content between said glycines of said triads is less than 60 number %,
having as a motif present at least twice, either contiguous or non-contiguous, a sequence having the following formula:
{(GXO)ₙZ}ₘ
wherein:
X and O are selected such that the motif has a proline content of less than or equal to 40 number %;
Z has from 0 to 50 amino acids and is other than repetitive GXO;
m is at least 1;
n is in the range 4 to 100;
the total number of different triads in the (GXO)ₙ motif is at least three;
and said polymer has a collagen-like property selected from the group consisting of helix formation, reversible denaturation and gel formation.

2. A collagen-like polymer according to claim 1, wherein said polymer is further **characterized by**:
comprising an amino acid sequence Z and repetitive units (GUB), wherein said amino acid sequence Z, said repetitive units (GUB) and said repetitive units (GXO)ₙ are linked in said polymer being represented by the following formula:
(GU^{u}B^{b})ₚ{(GX^{x}O^{o})ₙ(GU^{u}B^{b})ₚ¹Z^{z}}ₘ¹
wherein:
Z has from 0 to 50 amino acids and is other than repetitive GXO;
z is in the range of 1 to m¹ and indicates that there are up to and including that number of different Z groups present;
U and B are any amino acids;
u and b indicate the U and B in each triad may be the same or different;
x and o indicate the X and O in each triad may be the same or different;
p and p¹ may be the same or different and are in the range of 1 to 6, except that p may also be 0;
m¹ is in the range of 1 to 20; and
n is not more than 100.

3. A collagen-like polymer according to claim 2, wherein said polymer is further **characterized by**
comprising an amino acid sequence J, wherein said amino acid J, said amino acid sequence Z, said repetitive units (GUB) and said repetitive units (GXO)ₙ are linked in said polymer being represented by the following formula:
J¹ ᵣ[(GU^{u}B^{b})ₚ{(GX^{x}O^{o})ₙ(GU^{u}B^{b})ₚ¹Z^{z}}ₘ¹]J² ᵣ
wherein:
J¹ and J² are the same or different and are amino acid sequences of from about 1 to 50 amino acids; and
the r's are the same or different and are 0 or 1.

4. A collagen-like polymer according to any preceding claim, wherein said polymer comprises at least 75% of triads having glycine as the first amino acid.

5. A collagen-like polymer according to any preceding claim, wherein said polymer comprises at least 10% proline of the amino acids and is not more than about 150 kD.

6. A collagen-like polymer according to any preceding claim, wherein at least one GXO consists of GAP, GPA, GPP, GAS, GPS, or GDP, wherein G is glycine, A is alanine, P is proline, S is serine, and D is aspartic acid.

7. A DNA sequence encoding a collagen-like polymer according to any preceding claim.

8. A DNA sequence comprising:
a gene encoding a collagen-like polymer according to any one of claims 1-6;
a transcriptional initiation regulatory region functional in a unicellular microorganism at the 5' end of said gene; and
a transcriptional termination regulatory region functional in a unicellular microorganism at the 3' end of said gene;
wherein said gene is under the transcriptional regulation of said regulatory regions.

9. A unicellular microorganism comprising a DNA sequence according to either claim 7 or claim 8.

10. A method for producing a collagen-like polymer according to any preceding claim, said method comprising the steps of:
growing a unicellular microorganism according to claim 9 for sufficient time for said collagen-like polymer to be expressed; and
isolating said collagen-like polymer.

11. A formed object comprising a collagen-like polymer according to any preceding claim.

12. A formed object according to claim 11, wherein said formed object is a gel, film or fiber.

## Patentansprüche

1. Collagenähnliches Polymer mit zumindest 30 kD, wie durch SDS-PAGE bestimmt, erhältlich durch Expression in einem einzelligen Organismus aus einem in vitro hergestellten Konstrukt,
umfassend zumindest 60 Gew.-% Triaden mit Glycin als erster Aminosäure und zahlenmäßig zumindest 40 %, wobei die Triaden zumindest ein Prolin umfassen, worin der zahlenmäßige Gesamt-Prolingehalt zwischen den Glycinen der Triaden weniger als 60 % beträgt,
das als zumindest zweimalig, entweder zusammenhängend oder nicht zusammenhängend, vorhandenes Motiv eine Sequenz der folgenden Formel aufweist:
{(GXO)ₙZ}ₘ
worin:
X und O so gewählt sind, dass das Motiv einen zahlenmäßigen Prolingehalt von ≤ 40 % aufweist;
Z 0 bis 50 Aminosäuren aufweist und keine Wiederholung von GXO ist;
m zumindest 1 ist;
n im Bereich von 4 bis 100 liegt;
die Gesamtanzahl unterschiedlicher Triaden im (GXO)ₙ-Motiv zumindest 3 beträgt; und
das Polymer eine collagenähnliche Eigenschaft aufweist, die aus der aus Helixbildung, reversibler Denaturierung und Gelbildung bestehenden Gruppe ausgewählt ist.

2. Collagenähnliches Polymer nach Anspruch 1, worin das Polymer weiters **dadurch gekennzeichnet ist, dass**
es eine Aminosäuresequenz Z und repetitive Einheiten (GUB) umfasst, worin die Aminosäuresequenz Z, die repetitiven Einheiten (GUB) und die repetitiven Einheiten (GXO)ₙ im Polymer verbunden sind, das durch die folgende Formel dargestellt ist:
(GU^{u}B^{b})ₚ{(GX^{x}O^{o})ₙ(GU^{u}B^{b})ₚ¹Z^{z}}ₘ¹
worin:
Z 0 bis 50 Aminosäuren aufweist und keine Wiederholung von GXO ist;
z im Bereich von 1 bis m¹ liegt und anzeigt, dass maximal diese Anzahl an unterschiedlichen Z-Gruppen vorhanden ist;
U und B beliebige Aminosäuren sind;
u und b anzeigen, dass U und B in jeder Triade gleich oder voneinander verschieden sein können;
x und o anzeigen, dass X und O in jeder Triade gleich oder voneinander verschieden sein können;
p und p¹ gleich oder voneinander verschieden sein können und im Bereich von 1 bis 6 liegen können, mit der Ausnahme, dass p auch 0 sein kann;
m¹ im Bereich von 1 bis 20 liegt; und
n nicht über 100 liegt.

3. Collagenähnliches Polymer nach Anspruch 2, worin das Polymer weiters **dadurch gekennzeichnet ist, dass** es
eine Aminosäuresequenz J umfasst, worin die Aminosäure J, die Aminosäuresequenz Z, die repetitiven Einheiten (GUB) und die repetitiven Einheiten (GXO)ₙ im Polymer verbunden sind, das durch die folgende Formel dargestellt ist:
J¹ ᵣ[(GU^{u}B^{b})ₚ{(GX^{x}O^{o})ₙ(GU^{x}B^{b})ₚ¹Z^{z}}ₘ¹J² ᵣ
worin:
J¹ und J² gleich oder voneinander verschieden sind und Aminosäuresequenzen aus etwa 1 bis 50 Aminosäuren sind; und
die r gleich oder voneinander verschieden sind und 0 oder 1 sind.

4. Collagenähnliches Polymer nach einem der vorangegangenen Ansprüche, worin das Polymer zumindest 75 % Triaden mit Glycin als erster Aminosäure umfasst.

5. Collagenähnliches Polymer nach einem der vorangegangenen Ansprüche, worin das Polymer zumindest 10 % der Aminosäuren an Prolin umfasst und nicht mehr als etwa 150 kD aufweist.

6. Collagenähnliches Polymer nach einem der vorangegangenen Ansprüche, worin zumindest ein GXO aus GAP, GPA, GPP, GAS, GPS oder GDP besteht, worin G Glycin ist, A Alanin ist, P Prolin ist, S Serin ist und D Asparaginsäure ist.

7. DNA-Sequenz, die für ein collagenähnliches Polymer nach einem der vorangegangenen Ansprüche kodiert.

8. DNA-Sequenz, umfassend:
ein Gen, das für ein collagenähnliches Polymer nach einem der Ansprüche 1 bis 6 kodiert;
eine Transkriptionsinitiationsregulationsregion, die in einem einzelligen Mikroorganismus am 5'-Ende des Gens funktionell ist; und
eine Transkriptionsterminationsregulationsregion, die in einem einzelligen Mikroorganismus am 3'-Ende des Gens funktionell ist;
worin das Gen unter der Transkriptionsregulation der Regulationsregionen steht.

9. Einzelliger Mikroorganismus, der eine DNA-Sequenz nach einem der Ansprüche 7 oder 8 umfasst.

10. Verfahren zur Herstellung eines collagenähnlichen Polymers nach einem der vorangegangenen Ansprüche, wobei das Verfahren die folgenden Schritte umfasst:
das Züchten eines einzelligen Mikroorganismus nach Anspruch 9 für ausreichende Zeit, damit das collagenähnliche Polymer exprimiert wird; und
das Isolieren des collagenähnlichen Polymers.

11. Formgegenstand, der ein collagenähnliches Polymer nach einem der vorangegangenen Ansprüche umfasst.

12. Formgegenstand nach Anspruch 11, worin der Formgegenstand ein Gel, eine Folie oder eine Faser ist.

## Revendications

1. Polymère ressemblant à du collagène d'au moins 30 kD en déterminant par SDS-PAGE, pouvant être obtenu par expression dans un organisme unicellulaire à partir d'une construction préparée in vitro,
comprenant au moins 60% en poids de triades ayant de la glycine pour le premier acide aminé et au moins 40% en nombre desdites triades comprenant au moins une proline, où la teneur totale en proline entre lesdites glycines et desdites triades est inférieure à 60% en nombre,
ayant comme motif présent au moins deux fois, soit contigu ou non contigu, une séquence ayant la formule suivante :
{(GXO)ₙZ}ₘ
où :
X et O sont sélectionnés de façon que le motif ait une teneur en proline de moins de ou égale à 40% en nombre ;
Z a de 0 à 50 acides aminés et est autre que GXO répétitif ;
m est au moins 1 ;
n est dans la gamme de 4 à 100 ;
le nombre total de triades différentes dans le motif (GXO)ₙ est d'au moins trois ;
et ledit polymère a une propriété ressemblant au collagène, sélectionnée dans le groupe consistant en formation en hélice, dénaturation réversible et formation d'un gel.

2. Polymère ressemblant au collagène selon la revendication 1, où ledit polymère est de plus **caractérisé en ce que** :
il comprend une séquence d'acides aminés Z et des unités répétitives (GUB), où ladite séquence d'acides aminés Z, lesdites unités répétitives (GUB) et lesdites unités répétitives (GXO)ₙ sont enchaînées dans ledit polymère qui est représenté par la formule suivante :
(Gu^{u}B^{b})ₚ{(GX^{x}O^{o})ₙ(GU^{u}B^{b})ₚ¹Z^{z}}ₘ¹
où :
Z a de 0 à 50 acides aminés et est autre que GXO répétitif ;
z est dans la gamme de 1 à m¹ et indique qu'il y a jusqu'à et y compris le nombre de groupes Z différents présents ;
U et B sont tout acide aminé ;
u et b indiquent que U et B dans chaque triade peuvent être identiques ou différents ;
x et o indiquent que X et O dans chaque triade peuvent être identiques ou différents ;
p et p¹ peuvent être identiques ou différents et sont dans la gamme de 1 à 6, à l'exception que p peut également être 0 ;
m¹ est dans la gamme de 1 à 20 ; et
n n'est pas supérieur à 100.

3. Polymère ressemblant à du collagène selon la revendication 2, où ledit polymère est de plus **caractérisé en ce que**
il comprend une séquence d'acides aminés J, où ledit acide aminé J, ladite séquence d'acides aminés Z, desdites unités répétitives (GUB) et lesdites unités répétitives (GXO)ₙ sont enchaînés dans ledit polymère qui est représenté par la formule suivante :
J¹ ᵣ[(Gu^{u}B^{b})ₚ{(GX^{x}O^{o})ₙ(GU^{u}B^{b})ₚ¹Z^{z}}ₘ¹]J² ᵣ
où :
J¹ et J² sont identiques ou différents et sont deux séquences d'acides aminés d'environ 1 à 50 acides aminés ; et
Les r sont identiques ou différents et sont 0 ou 1.

4. Polymère ressemblant à du collagène selon toute revendication précédente, où ledit polymère comprend au moins 75% de triades ayant de la glycine pour le premier acide aminé.

5. Polymère ressemblant à du collagène selon toute revendication précédente, où ledit polymère comprend au moins 10% de proline des acides aminés et n'a pas plus d'environ 150 Kd.

6. Polymère ressemblant à du collagène selon toute revendication précédente, où au moins un GXO consiste en GAP, GPA, GPP, GAZ, GPS ou GDP, où G est glycine, A est alanine, P est proline, S est sérine et D est acide aspartique.

7. Séquence d'ADN codant pour un polymère ressemblant à du collagène selon toute revendication précédente.

8. Séquence d'ADN comprenant :
un gène codant pour un polymère ressemblant à du collagène selon l'une quelconque des revendications 1-6 ;
une région de régulation d'initiation de transcription fonctionnelle dans un microorganisme unicellulaire à l'extrémité 5' dudit gène ; et
une région de régulation de terminaison de transcription fonctionnelle dans un microorganisme unicellulaire à l'extrémité 3' dudit gène ;
où ledit gène est sous la régulation de transcription desdites régions de régulation.

9. Microorganisme unicellulaire comprenant une séquence d'ADN selon l'une quelconque des revendications 7 ou 8.

10. Méthode de production d'un polymère ressemblant à du collagène selon toute revendication précédente, ladite méthode comprenant les étapes de :
faire croître un microorganisme unicellulaire selon la revendication 9 pendant un temps suffisant pour que ledit polymère ressemblant à du collagène soit exprimé ; et
isoler ledit polymère ressemblant à du collagène.

11. Objet formé comprenant un polymère ressemblant à du collagène selon toute revendication précédente.

12. Objet formé selon la revendication 11, où ledit objet formé est un gel, un film ou une fibre.
